(19) Europäisches Patentamt

European Patent Office

Office européen des brevets

(11) **EP 1 273 663 A2**

(12) **EUROPEAN PATENT APPLICATION**

(43) Date of publication:
08.01.2003 Bulletin 2003/02

(51) Int Cl.[7]: **C12P 7/40**, C12P 7/44, C12N 15/53, C12N 1/15

(21) Application number: 02077029.3

(22) Date of filing: 20.07.1998

(84) Designated Contracting States:
**DE DK ES FR GB IE IT NL SE**

(30) Priority: **21.07.1997 US 53215 P**

(62) Document number(s) of the earlier application(s) in accordance with Art. 76 EPC:
**98935806.4 / 1 003 881**

(71) Applicant: **E.I. DUPONT DE NEMOURS AND COMPANY**
**Wilmington, Delaware 19898 (US)**

(72) Inventors:
• **Fallon, Robert D.**
**Elkton, Maryland 21921 (US)**

• **Payne, Mark S.**
**Wilmington, Delaware 19808 (US)**
• **Picataggio, Stephen K.**
**Landenberg, Pennsylvania 19350 (US)**
• **Wu, Shijun**
**New Castle, Delaware 19720 (US)**

(74) Representative: **Jones, Alan John et al**
**CARPMAELS & RANSFORD**
**43 Bloomsbury Square**
**London, WC1A 2RA (GB)**

Remarks:
This application was filed on 23 - 05 - 2002 as a divisional application to the application mentioned under INID code 62.

(54) **Transformed yeast strains and their use fore the production of monoterminal and diterminal aliphatic carboxylates**

(57) The present invention provides a bioprocess for converting aliphatic compounds of the formula $CH_3(CH_2)_nCH_3$, wherein n = 4 to 20, to monoterminal and diterminal carboxylates using a transformed yeast *Pichia pastoris* characterised by a genetically-engineered alkane hydroxylating activity.

EP 1 273 663 A2

**Description**

FIELD OF THE INVENTION

The present invention is a bioprocess for the conversion of aliphatic compounds, of the form $CH_3(CH_2)_nCH_3$ where n=4 to 20, to monoterminal and diterminal carboxylates by genetically-engineered organisms. This invention also relates to yeast strains with enhanced alkane hydroxylating activity and/or gene disruptions in the β-oxidation pathway for the production of carboxylates.

BACKGROUND OF THE INVENTION

Diterminal carboxylates of aliphatic compounds of the form $HO(O)C(CH_2)_nC(O)OH$ where n=7 to 16 are useful as polymer intermediates (U.S. 4,767,828) and as anticorrosion compounds (JP 08113771). Monoterminal carboxylates of aliphatic compounds of the form $CH_3(CH_2)_nC(O)OH$ where n=7 to 16 can serve as intermediates for surfactants (US 4,863,619). These compounds can be produced from natural plant sources (Dale et al., *J. Sci. Food Agr.*, 6:162, (1955)), but purification of the compound generally results in a great deal of by-product waste. Synthetic routes to enriched forms of such compounds with reduced waste by-products would be commercially advantageous.

A number of yeasts are known to grow by metabolizing aliphatic compounds of the form $CH_3(CH_2)_nCH_3$ where n=4 to 20. See, for example, Klug et al. (*Adv. in Microbial Physiology,* 5:1-43, (1971)). In addition, Mauersberger et al. (Non-conventional Yeasts in Biotechnology. A Handbook, Klause Wolf (ed.), Springer-Verlag, Berlin (1996)) note that *Candida maltosa* can grow on aliphatic compounds of chain length $C_6$ to $C_{40}$. In all cases examined to date, growth on aliphatic compounds depends on specific enzymatic steps which convert one or both terminal methyl groups to the carboxylate form. The first step in such transformations is the hydroxylation of the terminal methyl group by the yeast cytochrome P450 hydroxylating systems:

$$2CH_3(CH_2)_nCH_3 + O_2 + NADPH \rightarrow 2CH_3(CH_2)_nCH_2OH + NAD^+,$$

where n=7 to 16.

Such hydroxylating systems include at least three biological components: cytochrome P450 monooxygenase, cytochrome P450-NADPH reductase, and NADPH. The cytochrome P450-NADPH reductase transfers electrons from NADPH (or NADPH) to the cytochrome P450 monooxygenase, activating it. In the presence of oxygen and the aliphatic substrate, the activated cytochrome P450 catalyses the reaction between oxygen and the aliphatic substrate to form the corresponding alcohol. The necessity of electron transfer between the reductase and the cytochrome P450 monooxygenase requires proper structural orientation of the two components. In addition, the stoichiometric requirement for NADPH means that hydroxylating activity requires a continuous supply of NADPH. This NADPH supply is generally obtained from central metabolic pools in a living cell.

In general, the hydroxylated compound is further metabolized to the corresponding mono- or diterminal carboxylate which can then provide energy and carbon for yeast growth (Klug et al., *Adv. in Microbial Physiology,* 5:1-43, (1971)). The diploid yeast, *Candida maltosa,* can grow on alkanes as a sole carbon source by deriving its carbon and energy through the β-oxidation pathway. This pathway is so efficient that wild-type strains normally do not produce dicarboxylic acids via ω-oxidation during growth on alkanes. However, in some cases, the rate of carboxylate production exceeds the growth needs of the organism. Under the proper conditions, this excess carboxylate production is released into the growth medium. The resulting net production of carboxylates from aliphatic starting materials has been exploited for industrial production of enriched carboxylate liquors from which the desired carboxylate compounds can be easily separated.

The use of yeast for the production of mono- and diterminal carboxylates is known in the art. A variety of native ("wild type") strains have been exploited for diterminal acid production. US 4,275,158 discloses the use of *Debaryomyces vanrijiae* ATCC 20588 for the production of $C_{10}$ to $C_{18}$ diterminal carboxylates from aliphatic hydrocarbons or fatty acids. US 4,220,720 reports the use of *Debaryomyces phaffi* ATCC 20499 for a similar purpose. The use of other native strains is also reported for such carboxylate production including production of diterminal carboxylates from $C_9$ to $C_{19}$ aliphatic hydrocarbons by *Pichia polymorpha* (JP 70024392) and production of carboxylates by *Candida cloacae* (JP 76006750).

The diterminal carboxylates produced through fermentation by most yeasts, including *Candida maltosa*, are most often shorter than the original aliphatic substrate by one or more pairs of carbon atoms and mixtures are common (Ogino et al., *Agr. Biol. Chem.* 29:1009-1015 (1965); Shiio et al., *Arg. Biol. Chem.* 35:2033-2012 (1971); Hill et al., *Appl. Microbial. Biotechnol*. 24:168-174(1986)). Chain shortening is due to the degradation of the substrate and product, after activation to their corresponding acyl-CoA ester, by the peroxisomal β-oxidation pathway. The initial step in the β-oxidation (fatty acid) pathway involves oxidation of the acyl-CoA ester to its enoyl-CoA, and is catalyzed by acyl-CoA oxidase. The enoyl-CoA is further metabolized to the β-ketoacyl-CoA by the action of enoyl-CoA hydratase and β-hydroxyacyl-CoA dehydrogenase. The fourth and last step of the β-oxidation pathway is catalyzed by acyl-CoA acetyltransferase (more commonly called acyl-CoA thiolase), which promotes reaction of the β-ketoacyl-CoA with a molecule of free coenzyme A to hydrolyze the carboxy-terminal two carbon fragment of the original fatty acid as acetyl-CoA.

Genetic mutations causing partial blockage of these latter reactions result in the formation of unsaturated or hydroxylated byproducts (Meussdoerffer et al., *Proc. - World Conf. Biotechnol. Fats Oils Ind.*, 142-147 (1988)). These undesirable byproducts are often associated with biological production of diterminal carboxylates. Mutants produced by classical mutagenisis or by genetic engineering that enhance carboxylate production in excess of growth needs have also been reported in the art. Mutants of *Candida lipolytica* (EP 229252, DE 3929337, DE 4019166), *Candida tropicalis* (DE 3929337, DE 4019166, EP 296506, EP 316072, US 5,254,466), *Pichia carbofelas* (JP 57129694), *Torulopsis candida* (JP 52018885) and *Torulopsis bombicola* (US 3,796,630) have been described. Enhancement of excess carboxylate production has been achieved in these cases by decreasing the ability of the yeast to consume the desired carboxylate as part of its normal metabolism. Often, mutants partially defective in their ability to grow on alkane, fatty acid or di-carboxylic acid substrates demonstrate enhanced di-carboxylic acid yields. However, most mutants have not been characterized beyond their reduced ability to use these compounds as a carbon source for growth. In all likelihood, their ability to produce diterminal carboxylates is enhanced by a partial blockage of the β-oxidation pathway. Furthermore, compounds known to inhibit β-oxidation (i.e., acrylate) also result in increased diterminal carboxylate yields.

In regards to a biocatalyst for producing carboxylates, it would be desirable to have an effective block of the β-oxidation pathway at its first reaction, catalyzed by acyl-CoA oxidase. A complete block at this step, would result in enhanced yields of diterminal carboxylates by redirecting the substrate toward the ω-oxidation pathway while preventing reutilization of the diterminal carboxylate products through the β-oxidation pathway. In addition, the use of such a mutant would prevent the undesirable chain modifications associated with the β-oxidation pathway, such as unsaturation, hydroxylation, or chain shortening. With *Candida maltosa,* the β-oxidation pathway may be functionally blocked by inactivation of both POX4 genes, which encode acyl-CoA oxidase (Masuda et al., *Gene,* 167:157-161 (1995), in order to redirect the metabolic flux to the microsomal ω-oxidation pathway and thereby increase the yield of desired carboxylates.

A method for targeted gene disruption in yeast of the genus *Pichia* has been disclosed in EP 226752. In addition, US 5,254,466 claims a method of complete blockage of carboxylate consumption through genetic engineering of *Candida tropicalis.* There is a great deal of scientific evidence to support the vast difference between *Candida tropicalis* and *Candida maltosa* as a commercial biocatalyst, *infra*. Furthermore, a number of strains of *Candida maltosa* that metabolize aliphatic hydrocarbons for growth have been described (Bos et al., *Antoni van Leeuwenhoek,* 39: 99-107, (1973)). However, the prior art does not report the use of *Candida maltosa* for production of mono- or diterminal carboxylates.

In addition to blockage of the β-oxidation pathway, recently another strategy has been reported as a possible route to enhancement of excess carboxylate production in yeasts. Rather than inhibiting consumption, attempts have been made to enhance carboxylate production through enhancement of cytochrome P450 hydroxylating activity. DE 19507546 discloses expression of alkane hydroxylating cytochrome P450 systems in *Saccharomyces cerevisiae*, a yeast normally not capable of aliphatic hydrocarbon or fatty acid hydroxylation. Enhanced alkane cytochrome P450 monooxygenase activity in this yeast naturally results in carboxylate accumulation where the normal pathways for rapid carboxylate consumption are lacking. However, cytochrome P450 monooxygenase activity in this strain appears unusually sensitive to poisoning by oxygen (Zimmer et al., *DNA & Cell Biology,* 14:619-628, (1995)), perhaps indicating that the necessary structural integrity is lacking in this genetically-engineered strain.

WO 9114781 recites methods for the amplification of cytochrome P450 hydroxylating systems through genetic engineering in *Candida tropicalis*. Although some enhancement of carboxylate production was observed, the cytochrome P450 enzyme was poorly expressed and improvements in activity were not completely successful (Picataggio et al., *Bio/Technology*, 10:894-898, (1992)). In addition, German patent DE 3929337 describes the limited success of selection of mutants with improved cytochrome P450 monooxygenase activity and dicarboxylate production through the use of the selective agent, 1-dodecyne.

Wild-type *Candida maltosa* strains IAM12247 and ATCC 28140 are equivalent organisms. They are available from the Institute of Applied Microbiology (The University of Tokyo, Tokyo, Japan) and the American Type Culture Collection (Manassas, VA, USA), respectively. Strains ATCC 90625 and 90677 are derived from IAM12247 and contain the nutritional marker mutations ade1, his5 (90625) and ade1, his5, ura3 (90677). Both of these strains are available from the American Type Culture Collection, 1995 Yeast Reference Guide, 19th ed.

Recent reports have described DNA sequence information for a number of alkane cytochrome P450 monooxygenase as well as for the cytochrome P450 reductase from *Candida maltosa* IAM12247 / ATCC 28140 (Ohkuma et al., *DNA & Cell Biology,* 14:163-173, (1995); Kargel et al., *Yeast,* 12:333-348, (1996)). At least eight structurally distinct cytochrome P450s with different substrate specificities have been identified for *Candida maltosa.* Each of these integral membrane proteins requires electron transfer from NADPH via a cytochrome P450-NADPH reductase to catalyze monooxygenase reactions.

Mutated marker strains such as these are commonly used for genetic transformations. However, in light of the limited success reported to date for homologous expression of P450 monooxygenase systems in *Candida tropicalis,* it has been uncertain whether such biocatalysts can be developed in *Candida maltosa.*

There is a great deal of evidence that a biocatalyst for dodecanedioic acid (DDDA) production based on *Candida maltosa* will be distinctly different than one based on *Candida tropicalis.* These are two distinct species in the field of yeast taxonomy and significant differences exist between the two species at the molecular and biochemical level (Meyer et al., *Arch. Microbiol.*, 104:225-231 (1975)). These distinctions, besides their taxonomic importance, have practical implications.

*Candida maltosa* cannot grow on starch. *Candida tropicalis* can grow on starch. *Candida maltosa* is generally resistant to high concentrations of cyclohexamide while *Candida tropicalis* is not. *Candida tropicalis* is often associated with human disease while *Candida maltosa* is not.

These differences affect the utility of the organisms as a biocatalyst in industrial processes. Starch is an inexpensive source for slow glucose release and a promising co-substrate for DDDA production by *Candida tropicalis.* Starch is not a co-substrate option for *Candida maltosa. Candida maltosa* insensitivity to cyclohexamide eliminates use of one of the few antibiotic selection techniques available for yeast genetic engineering.

Molecular comparisons also distinguish the two species from one another. One widely accepted approach to evaluate evolutionary distances between species is based on DNA sequence comparisons for the small ribosomal RNA subunit (18S). To date, comparisons between *Candida maltosa* and *Candida tropicalis* have shown high similarities in these sequences, i.e., ≥94% (Ohkuma et al., *Biosci. Biotech. Biochem.,* 57:1793-1794 (1993)); Pesole et al., *Genetics,* 141:903-907 (1995); Cai et al., *Internal. J. Sys. Bacteriol.,* 46:542-549 (1996)). However, comparisons of GenBank sequences for key enzymes in the alkane oxidation process (cytochrome P450 monooxygenases and cytochrome P450 reductase) show greater dissimilarities for these genes than for the 18S RNA gene comparisons. For cytochrome P450 reductase, DNA sequences similarity equals only 83%. For cytochromes P450 monooxygenase, maximum DNA sequence similarity in a 7 gene by 7 gene comparison is only 77%. The majority of cytochromes P450 monooxygenase sequence comparisons are below 70%. This suggests that the genes important to the alkane oxidation process are under selective pressure and have evolved separately in these two distinct species. In fact, Meyer et al. *(Arch. Microbiol.,* 104:225-231 (1975)) have noted that the two species appear to occupy distinct ecological niches. *Candida maltosa* is only found in hydrocarbon-contaminated environments. *Candida tropicalis* is most often found associated with warm blooded animals, although it can grow in hydrocarbon-contaminated environments. Finally, total genomic DNA reassociation experiments show that *Candida maltosa* and *Candida tropicalis* share <40% total DNA similarity (Meyer et al., *Arch. Microbiol.,* 104:225-231 (1975)). Such differences in the molecular biology of *Candida maltosa* and *Candida tropicalis* make it uncertain whether genes from the two organisms will behave in a similar manner. Thus, limited success in causing enhanced P450 system activity in *Candida tropicalis* does not assure success in enhancing activity in *Candida maltosa.* Enhanced homologous expression of some P450 genes has been demonstrated (Ohkuma et al., *Biochim. Biophys. Acta.*, 1236:163-169 (1995)), but there have been no reports of enhanced P450 monooxygenase activity in *Candida maltosa*.

Successful expression of active P450 monooxygenase systems in genetically-engineered *Candida maltosa* could lead to useful biocatalysts for carboxylate production. To date, no report of a *Candida maltosa* transformant capable of a combined expression of alkane P450 monooxygenase, fatty acid monooxygenase and cytochrome P450-NADPH reductase expression is known in the art.

SUMMARY OF THE INVENTION

The present invention relates to a process for the bioproduction of $C_6$ to $C_{22}$ mono- and di-carboxylic acids by contacting, under aerobic conditions, transformed *Pichia pastoris* characterized by a genetically engineered enhanced alkane hydroxylating activity with at least one $C_6$ to $C_{22}$ straight chain hydrocarbon in the form $CH_3(CH_2)_nCH_3$ wherein n=4 to 20.

Another embodiment of the invention is a process for bioproduction of $C_6$ to $C_{22}$ mono- and di-carboxylic acids by contacting, under aerobic conditions, transformed *Candida maltosa* characterized by a genetically engineered enhanced alkane hydroxylating activity with at least one $C_6$ to $C_{22}$ straight chain hydrocarbon in the form $CH_3(CH_2)_nCH_3$ where n=4 to 20.

A further embodiment of the invention is a transformed *Pichia pastoris* comprising at least one foreign gene encoding a cytochrome P450 monooxygenase and at least one foreign gene encoding a cytochrome P450 reductase, each gene operably linked to suitable regulatory elements such that alkane hydroxylating activity is enhanced. The genes encoding cytochrome P450s are selected from the group consisting of P450 Alk1-A (D12475), Alk2-A (X5881), Alk3-A (X55881), Alk4-A (D12716), Alk5-A (D12717), Alk6-A (D12718), Alk7 (D12719) and Alk8 (D12719) or genes substantially similar thereto.

An additional embodiment of the invention is a transformed *Candida maltosa* comprising at least one additional copy of genes encoding cytochrome P450 monooxygenases and/or at least one additional copy of genes encoding cytochrome P450 reductase, wherein the genes are operably linked to suitable regulatory elements, such that alkane hydroxylating activity is enhanced. Additionally, the instant invention describes the construction of expression cassettes designed to deregulate expression of the major alkane monooxygenase (P450Alk1-A), fatty acid monooxygenase (P450Alk3-A) and cytochrome P450-NADPH reductase by precise fusion to the *Candida maltosa* phosphoglycerol kinase (PGK) promoter and terminator.

The instant invention relates to a process for bioproduction of $C_6$ to $C_{22}$ mono- and diterminal carboxylates by contacting, under aerobic conditions, transformed *Candida maltosa* characterized by a genetically-engineered, blocked β-oxidation pathway with at least one $C_6$ to $C_{22}$ straight chain hydrocarbon in the form $CH_3(CH_2)_nCH_3$ where n=4 to 20.

A further embodiment to the invention relates to a process for bioproduction of $C_6$ to $C_{22}$ mono- and diterminal carboxylates by contacting, under aerobic conditions, transformed *Candida maltosa* characterized by a genetically-engineered, blocked β-oxidation pathway and enhanced alkane hydroxylating activity with at least one $C_6$ to $C_{22}$ straight chain hydrocarbon in the form $CH_3(CH_2)_nCH_3$ where n=4 to 20.

An additional embodiment of the invention is genetically-engineered *Candida maltosa* strains that have enhanced cytochrome P450 activity and/or gene disruptions in the β-oxidation pathway.

A further embodiment of the invention is in novel DNA fragments. These fragments comprise (a) a first *Candida maltosa* promoter operably linked to a DNA encoding at least one polypeptide from *Candida maltosa* and (b) a second *Candida maltosa* promoter operably linked to a DNA encoding at least one polypeptide from *Candida maltosa.* The gene linked to the first *Candida maltosa* promoter encodes cytochrome P450 monooxygenase and the gene linked to the second *Candida maltosa* promoter encodes cytochrome P450 reductase. More preferably, the first *Candida maltosa* promoter is PGK, the gene encoding cytochrome P450 monooxygenase is Alk1-A (D12475), Alk2-A (X55881), Alk3-A (X55881), Alk4-A (D12716), Alk5-A (D12717), Alk6-A (D12718), Alk7 (D12719), and Alk8 (D12719).

BRIEF DESCRIPTION OF THE SEQUENCE DESCRIPTIONS. BIOLOGICAL DEPOSITS, AND FIGURES

The invention can be more fully understood from the following detailed description, the biological deposits, sequence descriptions, and Figures which form a part of this application.

Applicants have provided sequence listings in conformity with 37 C.F.R. §1.821-1.825 ("Requirements for Patent Applications Containing Nucleotide Sequences and/or Amino Acid Sequence Disclosures - the Sequence Rules") and consistent with World Intellectual Property Organization (WIPO) Standard ST.25 (1998) and the PCT and EPO sequence listing requirements.

SEQ ID NO:1 represents the sense primer for the cytochrome P450-NADPH reductase.

SEQ ID NO:2 represents the antisense primer for the cytochrome P450-NADPH reductase.

SEQ ID NO:3 represents the sense primer for the cytochrome P450Alk1-A gene.

SEQ ID NO:4 represents the antisense primer for the cytochrome P450Alk1-A gene.

SEQ ID NO:5 represents the sense primer for the cytochrome P450Alk3-A gene.

SEQ ID NO:6 represents the antisense primer for the cytochrome P450Alk3-A gene.

SEQ ID NO:7 represents the sense primer for the PGK promoter.

SEQ ID NO:8 represents the antisense primer for fusion of the PGK promoter to the P450Alk1-A gene.

SEQ ID NO:9 represents the sense primer for the 5' end of the P450Alk1-A gene.

SEQ ID NO:10 represents the antisense primer for the 5' end of the P450Alk1-A gene.

SEQ ID NO:11 represents the sense primer for the 3' end of the P450Alk1-A gene.

SEQ ID NO:12 represents the antisense primer for the 3' end of the P450Alk1-A gene.

SEQ ID NO:13 represents the sense primer for fusion of the PGK terminator to the P450Alk1-A gene.

SEQ ID NO:14 represents the antisense primer for the PGK terminator.

SEQ ID NO:15 represents the antisense primer the fusion of for the PGK promoter to the P450Alk3-A gene.

SEQ ID NO:16 represents the sense primer for the 5' end of the P450Alk3-A gene.

SEQ ID NO: 17 represents the antisense primer for the 5' end of the P450Alk3-A gene.

SEQ ID NO:18 represents the sense primer for the 3' end of the P450Alk3-A gene.

SEQ ID NO:19 represents the antisense primer for the 3' end of the P450Alk3-A gene.

SEQ ID NO:20 represents the sense primer for fusion of the PGK terminator to the P450Alk3-A gene.

SEQ ID NO:21 represents the antisense primer for fusion of the PGK promoter to the cytochrome P450-ADPH reductase gene.

SEQ ID NO:22 represents the sense primer for the 5' end of the cytochrome P450-NADPH reductase gene.

SEQ ID NO:23 represents the antisense primer for the 5' end of the cytochrome P450-NADPH reductase gene.

SEQ ID NO:24 represents the sense primer for the 3' end of the cytochrome P450-NADPH reductase gene.

SEQ ID NO:25 represents the antisense primer for the 3' end of the cytochrome P450-NADPH reductase gene.

SEQ ID NO:26 represents the sense primer for fusion of the PGK terminator to the cytochrome P450-NADPH reductase gene.

SEQ ID NO:27 represents the sense primer to the *Candida maltosa* POX4 gene.

SEQ ID NO:28 represents the antisense primer to the *Candida maltosa* POX4 gene.

SEQ ID NO:29 represents the sense primer to the *Candida maltosa* URA3 gene.

SEQ ID NO:30 represents the antisense primer to the *Candida maltosa* URA3 gene.

SEQ ID NO:31 represents the sense primer to the *Candida maltosa* ADE1 gene.

SEQ ID NO:32 represents the antisense primer to the *Candida maltosa* ADE1 gene.

SEQ ID NO:33 represents the sense primer to the *Candida maltosa* HIS5 gene.

SEQ ID NO:34 represents the antisense primer to the *Candida maltosa* HIS5 gene.

Applicants have made the following biological deposits under the terms of the Budapest Treaty on the International Recognition of the Deposit of Microorganisms for the Purposes of Patent Procedure. As used herein, "ATCC" refers to the American Type Culture Collection International Depository located at 10801 University Boulevard, Manassas, VA 20110-2209 U.S.A. The "ATCC No." is the accession number to cultures on deposit with the ATCC.

| Depositor Identification Reference | International Depository Designation | Date of Deposit |
|---|---|---|
| *Pichia pastoris* SW64/65 | ATCC 74409 | 3 April 1997 |
| *Candida maltosa* SW81/82 | ATCC 74431 | 10 December 1997 |
| *Candida maltosa* SW84/87.2 | ATCC 74430 | 10 December 1997 |

*Pichia pastoris* SW64/65 is characterized as a *Pichia pastoris* strain with the unusual ability that when induced by the presence of methanol is capable of producing active alkane cytochrome P450s which will convert $C_6$ to $C_{22}$ alkanes to the corresponding mono and diacids.

*Candida maltosa* SW81/82 is characterized as a *Candida maltosa* that is unusual in its inability to grow on $C_6$ to $C_{22}$ alkanes or monofatty acids and also is unusual in its ability to produce diacids from $C_6$ to $C_{22}$ monoacids or alkanes in the presence of suitable carbon and energy sources such as glycerol. This strain contains disrupted POX4 genes and has other auxotrophic markers removed. This strain is β-oxidation blocked.

*Candida maltosa* SW 84/87.2 is characterized as a *Candida maltosa* that is unusual in its inability to grow on $C_6$ to $C_{22}$ alkanes or monofatty acids and also is unusual in its ability to produce diacids from $C_6$ to $C_{22}$ monoacids or alkanes in the presence of suitable carbon and energy sources such as glycerol. In addition, SW84/87.2 is unusual in its ability to oxidize $C_6$ to $C_{22}$ alkanes or monoacids to diacids in the presence of glucose at greater than 5 g/L concentration. This strain expresses enchanced alkane hydroxylating activity and contains disrupted POX4 genes.

Figure 1 shows the strain lineage of β-oxidation-blocked *Candida maltosa* via the Southern blot of XmnI-digested genomic DNA probed with the POX4 gene.

Figure 2 is a restriction map of pSW83.

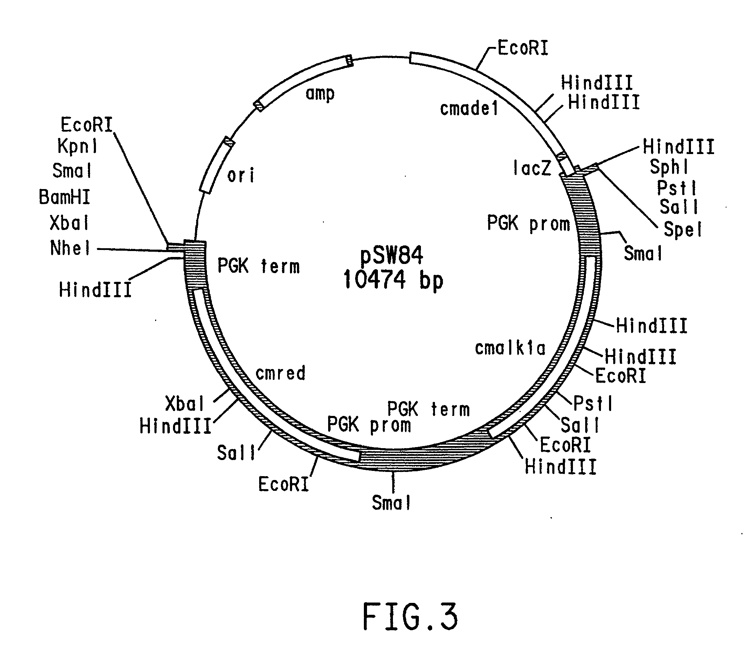

Figure 3 is a restriction map of pSW84.

Figure 4 is a restriction map of pSW85.

Figure 5 is a restriction map of pSW87.

DETAILED DESCRIPTION OF THE INVENTION

The present invention comprises a process for the bioconversion of aliphatic compounds, of the form $CH_3(CH_2)_nCH_3$ where n=4 to 20, to monoterminal and diterminal carboxylates using genetically-engineered organisms. The present invention describes for the first time transformed *Candida maltosa* strains that have enhanced cytochrome P450 activity (including combined, simultaneous expression of alkane P450 monooxygenase, fatty acid monooxygenase and cytochrome P450-NADPH reductase expression) and/or gene disruptions in the β-oxidation pathway. Based on growth and alkane utilization rates of the wild-type strain, further improvements in volumetric productivity (g product/L/hr) of either the P450 enhanced or β-blocked-strain would be required for an economical bioprocess. Hence, the combination of these two concepts provides a superior biocatalyst for the production of mono- and diterminal carboxylates from aliphatic substrates. The present invention gives the desired carboxylates in quantities and conversion efficiencies sufficient to be commercially viable.

One recombinant organism expresses enhanced alkane hydroxylating activity. The alkane hydroxylating activity is responsible for the hydroxylation of a terminal methyl group. The enhanced hydroxylating activity may be due to enhanced alkane monooxygenase, fatty acid monooxygenase or cytochrome P450 reductase separately or in various combinations. Additional enzymatic steps are required for its further oxidation to the carboxylate form. Two further oxidation steps, catalyzed by alcohol oxidase (Kemp et al., *Appl. Microbiol. and Biotechnol.,* 28:370 (1988)) and alcohol dehydrogenase, lead to the corresponding carboxylate.

Another recombinant organism has gene disruptions in the β-oxidation pathway. The diploid yeast, *Candida maltosa*, grows on alkanes as a sole carbon source by deriving its carbon and energy through the β-oxidation pathway. This pathway is so efficient that wild-type strains normally do not produce di-carboxylic acids via ω-oxidation during growth on alkanes. The β-oxidation pathway was blocked in order to increase the metabolic flux to the ω-oxidation pathway and thereby increase the yield and selectivity of a bioprocess for conversion of alkanes to mono- and diterminal carboxylates.

A third recombinant organism has both enhanced alkane hydroxylating activity and gene disruptions in the β-oxidation pathway. The enhanced hydroxylating activity may be due to enhanced alkane monooxygenase, fatty acid monooxygenase or cytochrome P450 reductase separately or in various combinations. The products of the present invention are useful as intermediates in the production of anticorrosive compounds and surfactants. More particularly, the methods and materials of the invention are useful for the bioproduction of dodecanedioic acid. The bioprocess provides improved flexibility in manufacturing and marketing of intermediates relative to the current chemical route to polymer-grade and chemical-grade dodecanedioic acid. Specifically, high yields with good selectivity can be obtained. Further, the commercial bioprocess is expected to effect the environment more favorably than does the current chemical process.

Terms and abbreviations used in this disclosure are defined as follows:

"Reduced nicotinamide-adenine dinucleotide" is abbreviated as NADPH.
"Reduced nicotinamide-adenine dinucleotide phosphate" is abbreviated as NADPH.
*"Candida maltosa* IAM12247 cytochrome P450Alk1-A gene" is abbreviated as Alk1-A.
"*Candida maltosa* IAM12247 cytochrome P450Alk3-A gene" is abbreviated as Alk3-A.
"*Candida maltosa* cytochrome P450-NADPH reductase gene" is abbreviated as P450 reductase or CPR.
"*Candida maltosa* acyl CoA gene" is abbreviated as POX4.
"*Candida maltosa* IAM12247 URA3 gene codes for the enzyme orotidine-5'-monophosphate decarboxylase" is abbreviated as URA3.
"Phosphoglycerol kinase" is abbreviated PGK.
"Alcohol oxidase I" is abbreviated as AOX1.
"Gas chromatography" is abbreviated as GC.
"Polymerase chain reaction" is abbreviated as PCR.
"Autonomously replicating sequences" is abbreviated as ARS.
"Dodecanedioic acid" is abbreviated as DDDA.

The term "genetically-engineered" refers to the formation of new combinations of heritable material by the insertion of nucleic acid molecules, produced or derived by whatever means outside the cell, into any virus, bacterial plasmid or other vector system so as to allow their incorporation into a host organism in which they are propagated and expressed to alter the phenotype of the host organism.

The term "transformation" refers to genetic engineering in which a nucleic acid fragment is transferred into the genome of a host organism, resulting in genetically stable inheritance. Host organisms containing the transferred nucleic acid fragments are referred to as "transgenic" or "transformed" organisms or transformants.

The term "nucleic acid" refers to complex compounds of high molecular weight occurring in living cells, the fundamental units of which are nucleotides linked together with phosphate bridges. Nucleic acids are subdivided into two types: ribonucleic acid (RNA) and deoxyribonucleic acid (DNA).

An "isolated nucleic acid fragment" is a polymer of RNA or DNA that is single- or double-stranded, optionally containing synthetic, non-natural or altered nucleotide bases. An isolated nucleic acid fragment in the form of a polymer of DNA may be comprised of one or more segments of cDNA, genomic DNA or synthetic DNA.

The term "cytochrome P450" refers to a widely distributed monooxygenase, active in many different biological hydroxylation reactions and one component of the cytochrome P450 hydroxylating system.

The term "cytochrome P450 reductase" refers to a widely distributed reductase, active in many different biological hydroxylation reactions and one component of the cytochrome P450 hydroxylating system.

The terms "blocked β-oxidation pathway" and "β-blocked" refer to gene disruptions that effectively eliminate acyl-CoA oxidase, the first enzyme in the β-oxidation pathway of a wild-type.

"Altered levels" refers to the production of gene product(s) in organisms in amounts or proportions that differ from that of normal, wild-type, or non-transformed organisms. Production may be more specifically described as "enhanced" or "decreased" relative to that of normal, wild-type, non-transformed organisms.

The term "enhanced" refers to an improvement or increase over an original observation or function. Enhanced alkane hydroxylating activity is associated with at least one additional copy of genes (relative to the wildtype) encoding cytochromes P450 monooxygenase and/or cytochrome P450-NADPH reductase.

The terms "cassette" and "gene cassette" refer to a number of nucleotide sequences which have been deliberately joined or combined in-vitro into a unique construction. An "expression cassette" specifically includes a promoter fragment, a DNA sequence for a selected gene product and a transcription terminator.

The terms "plasmid" and "cloning vector" refer to an extra chromosomal element usually in the form of circular double-stranded DNA molecules and often carrying genes which are not part of the central metabolism of the cell. Such elements may be autonomously replicating sequences, genome integrating sequences, phage sequences, linear or circular, of a single- or double-stranded DNA or RNA, derived from any source. The term "autonomously replicating sequence" refers to chromosomal sequences with the ability to allow autonomous replication of plasmids in yeasts.

The term "expression" refers to the transcription and stable accumulation of sense (mRNA) or antisense RNA derived from the nucleic acid fragment of the invention. Expression may also refer to translation of mRNA into a polypeptide. "Overexpression" refers to the production of a gene product in transgenic organisms that exceeds levels of production in normal or non-transformed organisms. "Co-suppression" refers to the production of sense RNA transcripts capable of suppressing the expression of identical or substantially similar foreign or endogenous genes (U.S. 5,231,020).

The term "mutation" refers to a chemical change in the DNA of an organism leading to a change in the genetic character of the organism. A strain exhibiting such a changed characteristic is termed a "mutant".

The term "oligonucleotide primer" refers to a short oligonucleotide that base-pairs to a region of single-stranded template oligonucleotide. Primers are necessary to form the starting point for DNA polymerase to produce complementary-stranded synthesis with single-stranded DNA.

The terms "restriction enzyme" and "restriction endonuclease" refer to an enzyme which catalyzes hydrolytic cleavage within a specific nucleotide sequence in double-stranded DNA.

The term "straight chain hydrocarbon" refers to aliphatic hydrocarbons, fatty acids, and esters of fatty acids of carbon number $C_6$ to $C_{22}$ containing 0, 1 or 2 double bonds in the carbon backbone. In addition, the term includes any of the straight chain compounds described above where one of the terminal carbons has been replaced by a phenyl group. Specific preferred hydrocarbons are nonane, decane, undecane, dodecane, tridecane, tetradecane, pentadecane, hexadecane, heptadecane, octadecane or any of the respective mono-carboxylic acids. Preferred are $C_{12}$-$C_{14}$ alkanes. Dodecane is especially preferred.

The term "alkane hydroxylating activity" refers to the ability of an organism, such as a yeast, to enzymatically hydroxylate the terminal methyl group of a straight-chain hydrocarbon using a cytochrome P450 hydroxylating system. The term "cytochrome P450 hydroxylating system" refers to a hydroxylating system composed of at least the following three biological components:

1) cytochrome P450 monooxygenase, 2) cytochrome P450-NADPH reductase and 3) reduced nicotinamide-adenine dinucleotide (NADPH) or reduced nicotinamide-adenine dinucleotide phosphate (NADPH).

"Gene" refers to a nucleic acid fragment that encodes a specific protein, including regulatory sequences preceding (5' non-coding sequences) and following (3' non-coding sequences) the coding sequence. "Native gene" refers to a gene as found in nature with its own regulatory sequences. "Chimeric gene" refers to any gene that is not a native gene, comprising regulatory and coding sequences that are not found together in nature. Accordingly, a chimeric gene may comprise regulatory sequences and coding sequences that are derived from different sources, or regulatory sequences and coding sequences derived from the same source, but arranged in a manner different than that found in nature. "Endogenous gene" refers to a native gene in its natural location in the genome of an organism. A "foreign" gene refers to a gene not normally found in the host organism, but that is introduced into the host organism by gene transfer. Foreign genes can comprise native genes inserted into a non-native organism, or chimeric genes. A "transgene" is a gene that has been introduced into the genome by a transformation procedure.

"Coding sequence" refers to a DNA sequence that codes for a specific amino acid sequence. "Suitable regulatory sequences" refer to nucleotide sequences located upstream (5' non-coding sequences), within, or downstream (3' non-coding sequences) of a coding sequence, and which influence the transcription, RNA processing or stability, or translation of the associated coding sequence. Regulatory sequences may include promoters, translation leader sequences, introns, and polyadenylation recognition sequences.

"Promoter" refers to a DNA sequence capable of controlling the expression of a coding sequence or functional RNA. In general, a coding sequence is located 3' to a promoter sequence. The promoter sequence consists of proximal and more distal upstream elements, the latter elements often referred to as enhancers. An "enhancer" is a DNA sequence which can stimulate promoter activity and may be an innate element of the promoter or a heterologous element inserted to enhance the level or tissue-specificity of a promoter. Promoters may be derived in their entirety from a native gene, or be composed of different elements derived from different promoters found in nature, or even comprise synthetic DNA segments. It is understood by those skilled in the art that different promoters may direct the expression of a gene in different tissues or cell types, or at different stages of development, or in response to different environmental conditions. Promoters which cause a gene to be expressed under most growth conditions at most times are commonly referred to as "constitutive promoters". New promoters of various types useful in plant cells are constantly being discovered; numerous examples may be found in the compilation by Okamuro and Goldberg, *(Biochemistry of Plants* 15: 1-82 (1989)). It is further recognized that since in most cases the exact boundaries of regulatory sequences have not been completely defined, DNA fragments of different lengths may have identical promoter activity.

The term "operably linked" refers to the association of nucleic acid sequences on a single nucleic acid fragment so that the function of one is affected by the other. For example, a promoter is operably linked with a coding sequence when it affects the expression of that coding sequence (i.e., that the coding sequence is under the transcriptional control of the promoter). Coding sequences can be operably linked to regulatory sequences in sense or antisense orientation.

"Mature" protein refers to a post-translationally processed polypeptide; i.e., one from which any pre- or propeptides present in the primary translation product have been removed. "Precursor" protein refers to the primary product of translation of mRNA; i.e., with pre- and propeptides still present. Pre- and propeptides may be but are not limited to intracellular localization signals.

Construction of Recombinant *Pichia pastoris*:

Another embodiment of this invention relates to the genetic engineering of *Pichia pastoris* to achieve expression of active P450 systems derived from a heterologous source. Expression cassettes are constructed to include a promoter, such as, but not limited to, the strong, methanol-inducible promoter of alcohol oxidase I (AOX1) fused to the Alk1-A gene (or alternatively to the Alk3-A or P450 reductase genes) followed by a transcriptional terminator (such as from AOX1). The expression cassettes are subcloned into vectors containing suitable transformation markers, such as, but not limited to, HIS4, ARG4, SUC2 or the *sh ble* gene which encodes Zeocin resistance (Invitrogen, San Diego, CA, USA). Sequential transformations of an appropriate strain of *Pichia pastoris* by established methods (U.S. 4,855,231) results in the integration of expression cassettes for genes into the *Pichia pastoris* genome. Transformants harboring multiple copies of the expression cassettes can be identified by a variety of methods such as, but not limited to, PCR and Southern blot analysis.

An alternative embodiment of engineering *Pichia pastoris* for expression of active P450 systems derived from a heterologous source entails subcloning multiple expression cassettes onto one or two plasmids. For example, the expression cassettes for Alk1-A and Alk3-A genes may be subcloned on one plasmid and the expression cassette for P450 reductase gene may be subcloned on a second plasmid; or expression cassettes for Alk1-A and P450 reductase genes may be subcloned on one plasmid and the expression cassette for Alk3-A gene may be subcloned on a second plasmid; or the expression cassettes for Alk3-A and P450 reductase genes may be subloned on one plasmid and the expression cassette for Alk1-A gene may be subcloned on a second plasmid; or the expression cassettes for Alk1-A and Alk3-A and P450 reductase genes may be subcloned on one plasmid. The plasmids are then used to sequentially or simultaneously transform a suitable *Pichia pastoris* host. Transformants harboring multiple copies of the expression cassettes can be identified by a variety of methods such as, but not limited to, PCR and Southern blot analysis.

A further embodiment of engineering *Pichia pastoris* for expression of active P450 systems derived from a heterologous source entails subcloning expression cassettes for Alk1-A, Alk3-A and P450 reductase genes on to replicating plasmids, individually or in multiple copies as described above for the integration plasmids. The replicating plasmids are then used to sequentially or simultaneously transform a suitable *Pichia pastoris* host. Transformants harboring multiple copies of the expression cassettes can be identified by a variety of methods such as, but not limited to, PCR and Southern blot analysis.

Engineered *Pichia pastoris* cells containing multiple copies of expression cassettes for Alk1-A, Alk3-A and P450 reductase genes are grown to saturation in minimal medium containing glycerol (or glucose) as the carbon source, followed by induction of AOX1 promoter by methanol. This results in high level production of the P450 system components and high hydroxylating activity. Aliphatic substrate may be added before, at the beginning of, or any time during induction, and after a suitable time, the medium is analyzed for carboxylates as described above.

PCR Amplification of Genomic DNA from *Candida maltosa*:

Oligonucleotide primers are prepared based on sequences available from GenBank (National Center for Biotechnology Information, Bethesda, MD, USA) for the *Candida maltosa* IAM12247 cytochromes P450 Alk1-A and Alk3-A, and cytochrome P450 reductase genes, accession numbers D12475, X55881, and D25327, respectively. Appropriate, unique restriction sites are designed into the primers to allow convenient ligation into a cloning vector as well as construction of a gene expression cassette (See, for example, Sambrook et al., Molecular Cloning: A Laboratory Manual, Second Edition, Cold Spring Harbor Laboratory Press, (1989)). In a similar manner, oligonucleotide primers are designed for the *Candida maltosa* IAM12247 URA3 gene. Using polymerase chain reaction (PCR) U.S. Patent 4,683,202 (1987, Mullis et al.), and U.S. Patent 4,683,195 (1986, Mullis et al.), appropriate DNA sequences are amplified from genomic DNA obtained from *Candida maltosa* IAM12247 which corresponds to ATCC 90677. Similar protocols and appropriate primers would also allow PCR amplification of other *Candida maltosa* IAM12247 sequences available from GenBank including, but not limited to, cytochromes P450 Alk2-A (X5881), Alk4-A (D12716), Alk5-A (D12717), Alk6-A (D12718), Alk7 (D12719) and Alk8 (D12719).

Construction of Recombinant *Candida maltosa* - Chromosomal Integration:

The descriptions that follow are embodiments of the invention that use integrative transfer of the genes of interest in the transformed host.

The DNA fragments synthesized by PCR are sequentially inserted into a convenient cloning vector such as pUC18 or lambda Zap (Invitrogen, San Diego, CA, USA) producing a vector which includes the gene cassette of the form Alk1-A/Alk3-A/P450 reductase/URA3/Alk1-A. After cloning of the vector containing the gene cassette in *E. coli,* the cassette fragment is linearized by cutting with appropriate restriction enzymes. *Candida maltosa* IAM12247 (corresponding to ATCC 28140) is transformed using techniques known in the art (Sambrook et al., *supra)* and transformants which have gained functional copies of the URA3 gene are selected by growth on minimal medium supplemented with histidine and adenine sulfate. Genomic DNA is isolated from the transformed strains using techniques known in the art. The genomic DNA is cut using appropriate restriction enzymes followed by probing using the Southern blot method. In this way, clones that have the maximum number of gene copies inserted into the chromosome are determined. Higher gene copy number generally results in higher levels of enzyme activity.

A further embodiment of the invention is the sequential addition of the P450 system genes to the *Candida maltosa* chromosome. Insertion into the host genome of any cassette of the form X/URA3/X, where X = Alk1-A Alk3-A, P450 reductase genes or other P450 system genes described above is accomplished by following a similar protocol of PCR amplification, cloning, linearization, transformation, minimal medium selection, and Southern blot screening to produce clones containing at least one additional copy of gene X for each original copy in the chromosome. Since different cytochrome P450 enzymes may have different substrate specificities, the insertion of the genes Alk1-A Alk3-A and P450 reductase in any combination, or alternative insertions of one or more genes results in a set of biocatalysts useful for producing mono- or diterminal carboxylates from any appropriate substrate with a carbon number of 9 through 18.

The copy number of multiple genes is increased through successive integrative transformations, by inserting a recoverable marker gene along with the gene of interest during each transformation. In one embodiment of the invention, the URA3 gene is used repetitively. The ura3- genotype is regenerated by selective growth on 5-fluoroorotic acid after each transformation, allowing the same marker gene to be used for the next transformation. This process is repeated for each additional transformation. In another embodiment of the invention the *his5* (GenBank Accession No. X17310) or *ade1* (GenBank Accession No. D00855) marker genes are used as the marker gene. Since *Candida maltosa* strain ATCC 90677 is auxotropic for three different marker genes (URA3, HIS5 and ADE1), up to three genes of interest can be inserted before it is necessary to regenerate an auxotrophic mutation.

Construction of Recombinant *Candida maltosa* - Autonomous Replication:

In another embodiment of the invention an autonomously replicating sequence (ARS) is added to the vector containing a cassette having the genes encoding a cytochrome P450 system. The host *Candida maltosa* is transformed with this construct. The vector is stably maintained in the host as a result of the ARS and selection pressure on a medium lacking uracil. As a result of the extra copies of the genes of interest carried by the vector, expression of active P450 systems is increased resulting in greater carboxylate production. However, the invention should not be considered limited by the use of genes Alk1-A, Alk3-A, P450 reductase and URA3 in this example. Any of the P450 system genes identified in this strain of *Candida maltosa* could be included alone or in combination in a replicative plasmid construct and transformed into *Candida maltosa* for the creation of a useful biocatalyst. Of particular use in the present invention are the genes Alk1-A, Alk3-A and P450 reductase. As a result of increased expression levels of appropriate P450 system genes, higher levels of carboxylate are produced.

Reaction Conditions for *Candida maltosa*:

Clones containing the highest levels of cytochrome P450 hydroxylating activity are grown for 2-3 days on suitable medium, optionally containing effective amounts of aliphatic substrate. At the end of this period, additional substrate is added and the cells are incubated for another 1-2 days. Cells are removed and the supernatant is acidified resulting in the precipitation of the monoterminal and diterminal carboxylates. The precipitate and any dissolved carboxylates are extracted from the supernatant into methyl tertiary butyl ether (MTBE) and recovered in a substantially pure form after evaporation of the MTBE solvent.

Substrates for Reactions:

The use of dodecane as a substrate to produce carboxylates is included for illustrative purposes and should not be considered as limiting the scope of the invention. Alternative suitable substrates for carboxylate production include straight chain hydrocarbons of carbon number $C_6$ to $C_{22}$, alone or in combination. Fatty acids with carbon number $C_6$ to $C_{22}$ also serve as substrates for diterminal carboxylate production. Furthermore, aliphatic hydrocarbons or fatty acids containing 1 or 2 double bonds in the carbon backbone can serve as substrates for the production of carboxylates where one or two additional terminal carboxylate groups appear in the products. Any of the straight chain compounds described above where one of the terminal carbons has been replaced by a phenyl group are also useful for carboxylate production.

Cell Strains and Growth Conditions:

*Candida maltosa* strains ATCC 90625 and ATCC 90677 (see ATCC Catalogue of Yeasts) are used for transformation and expression of alkane hydroxylating activity. *Pichia pastoris* strain GTS115 is obtained from Invitrogen (San Diego, CA, USA). These strains are routinely grown in YEPD medium (yeast extract, 10 g/L; peptone, 20 g/L; glucose, 20 g/L) at 30 °C with shaking at 250 rpm. Transformants of *Candida maltosa* ATCC 90677 with additional functional copies of the URA3 gene are selected by growth on minimal medium supplemented with histidine and adenine sulfate. The minimal medium is YNB (DIFCO Laboratories, Detroit, MI, USA), with amino acids + 50 mg/L histidine and 20 mg/L adenosine sulfate + 10 g/L glucose.

GC Conditions:

The concentration of DDDA was determined by gas chromatography of the MSTFA+1% TMCS derivatives using a SE 54 capillary column (15 m x 0.53 mm), 1.2 µm coating with a temperature program of 1.5 min at 150 °C, 5 °C/min to 200 °C, 5 min at 200 °C; injector: 310 °C; detector: 320 °C; FID detection.

EXAMPLES

The present invention is further defined in the following Examples, in which all parts and percentages are by weight and degrees are Celsius, unless otherwise stated. It should be understood that these Examples, while indicating preferred embodiments of the invention, are given by way of illustration only. From the above discussion and these Examples, one skilled in the art can ascertain the essential characteristics of this invention, and without departing from the spirit and scope thereof, can make various changes and modifications of the invention to adapt it to various usage and conditions.

GENERAL METHODS

Procedures for enzymatic digestion of DNA with restriction endonucleases, phosphorylations, ligations, and transformations are well known in the art. Standard recombinant DNA and molecular cloning techniques used herein are well known in the art and are described more fully in Sambrook, J., Fritsch, E.F. and Maniatis, T. Molecular Cloning: A Laboratory Manual; Cold Spring Harbor Laboratory Press: Cold Spring Harbor (1989), and by Silhavy, T.J., Bennan, M.L., and Enquist, L.W. Experiments with Gene Fusions, Cold Spring Harbor Press: Cold Spring Harbor (1984), and by Ausubel, F.M. et al., Current Protocols in Molecular Biology, Greene Publishing Assoc. and Wiley-Interscience (1987).
Materials and methods suitable for the maintenance and growth of bacterial and yeast cultures are well known in the art. Techniques suitable for use in the following examples may be found in Manual of Methods for General Bacteriology (Phillipp Gerhardt, R. G. E. Murray, Ralph N. Costilow, Eugene W. Nester, Willis A. Wood, Noel R. Krieg and G. Briggs Phillips, eds), American Society for Microbiology, Washington, DC. (1994) or Thomas D. Brock in Biotechnology: A Textbook of Industrial Microbiology, Second Edition (1989) Sinauer Associates, Inc., Sunderland, MA. All reagents and materials used for the growth and maintenance of bacterial cells were obtained from Aldrich Chemicals (Milwaukee, WI, USA), DIFCO Laboratories (Detroit, MI, USA), GibcoBRL (Gaithersburg, MD, USA), or Sigma Chemical Company (St. Louis, MO, USA) unless otherwise specified.
The meaning of abbreviations is as follows: "h" means hour(s), "min" means minute(s), "sec" means second(s), "d" means day(s), "mL" means milliliters, "L" means liters, "µL" means microliter, and "mm" means millimeter(s).

EXAMPLE 1

Construction of *Pichia pastoris* strain expressing alkane hydroxylating activity

Cytochrome P450-NADPH reductase was PCR-amplified from *Candida maltosa* ATCC 90677 using primers 1 (SEQ ID NO:1) and 2 (SEQ ID NO:2) which incorporate terminal BamHI and AvrII sites (indicated in lower case letters), respectively:

Primer 1 - (SEQ ID NO:1):

5'-AggatccATGGCATTAGATAAATTAG-3'

Primer 2 - (SEQ ID NO:2):

5'-AcctaggCTACCAAACATCTTCTTG-3'

This DNA fragment was subcloned between the BamHI and AvrII sites of the vector pPIC3K (Invitrogen, San Diego, CA, USA) generating in pSW64 in which the AOXI promoter drives expression of the cytochrome P450-NADPH reductase gene. Cytochrome P450Alk1-A was PCR-amplified from *Candida maltosa* ATCC 90677 using primers 3 (SEQ ID NO:3) and 4 (SEQ ID NO:4) which incorporate terminal KpnI and ApaI sites (indicated in lower case letters), respectively.

Primer 3 - (SEQ ID NO:3):

5'-CggtaccATGGCTATAGAACAAATTA-3'

Primer 4 - (SEQ ID NO:4):

5'-AgggcccTTTAGCAGAAATAAACAC-3'

This DNA fragment was subcloned between the KpnI and ApaI sites of the vector pPICZA (Invitrogen, San Diego, CA, USA), generating pSW65 in which the AOXI promoter drives expression of the cytochrome P450Alk1-A gene. Cytochrome P450Alk3-A was PCR-amplified from *Candida maltosa* ATCC 90677 using primers 5 (SEQ ID NO:5) and 6 (SEQ ID NO:6) which incorporate terminal XhoI and ApaI sites (indicated in lower case letters), respectively.

Primer 5 - (SEQ ID NO:5):

5'-ActcgagATGCCGGTTTCCTTTGTTC-3'

Primer 6 - (SEQ ID NO:6):

5'-AgggcccGTACATTTGGATATTGG-3'

This DNA fragment was subcloned between the XhoI and ApaI sites of the vector pPICZA (Invitrogen, San Diego, CA, USA), generating pSW72 in which the AOXI promoter drives expression of the cytochrome P450 Alk3-A gene. The BglII/BamHI fragment from pSW72 containing the Alk3-A expression cassette was subcloned into the BamHI site of pSW65 which contains the Alk1-A expression cassette, generating pSW73 which contains expression cassettes for the Alk1-A and Alk3-A genes.

*Pichia pastoris* GTS115 *(his4)* was transformed with pSW64 to *HIS* prototrophy by the spheroplast method (Cregg et al., *Mol. Cell Biol.,* 5:3376-3385, (1985)) a step that integrates the plasmid into the genome. A high copy number transformant, designated SW64, was selected by growth in high concentration (>1 mg/mL) of G418 as described (Scorer et al., *Bio/Technology,* 12:181-184, (1994)). Strain SW64 was re-transformed with pSW65 to zeocin resistance by the electroporation method (Invitrogen, San Diego, CA, USA), a step that integrates the plasmid into the genome. PCR analysis verified the integration of expression cassettes for both P450 reductase and P450 Alk1-A genes into the genome of a double transformant, designated SW64/65 and identified by ATCC Accession No. 74409.

*Pichia pastoris* double transformant SW64/65 (ATCC 74409) was grown to saturation (48 h) in 20 mL MGY (1.34% yeast nitrogen base without amino acids, 1% glycerol, 0.00004% biotin) with shaking at 30 °C. Following centrifugation, cells were induced by resuspension in 20 mL MM+Fe (1.34% yeast nitrogen base without amino acids, 0.5% methanol, 0.00004% biotin, 1mM $Fe^{+3}$) and incubated with shaking at 30 °C for up to 48 h. Cells were washed twice in PBS (Sambrook et al., supra), once in sucrose buffer (0.25 M sucrose, 0.05 M Tris-HCl pH 7.5, 1 mM EDTA, 1 mM DTT), and resuspended in 2 mL sucrose buffer supplemented with 0.3% BSA (Sambrook et al., supra). Extract was prepared by vortexing cells with 1/2 volume of 0.5 mm glass beads (approx. 1 mL) for a total of 4 min in increments of 1 min, followed by 1 min on ice. The semi-clear lysate obtained by centrifugation (3000xg) of glass beads and cell debris was centrifuged at 12000xg. The resulting supernatant was centrifuged at 25000xg. The resulting supernatant was centrifuged at 45000xg, and the microsomal pellet was resuspended in 1 mL sucrose buffer supplemented with 0.3% BSA.

An equal volume (1 mL) of NADPH/NADPH mix (0.5 mM NADPH and 0.5 mM NADPH in sucrose buffer) was added to a microsome preparation. One μL of $^{14}$C-lauric acid (50mCi/mmole; ICN, Costa Mesa, CA, USA) was added and the mix incubated at 30 °C with shaking at 150 rpm for 0-60 min. The reaction was stopped by the addition of 0.1 mL $H_2SO_4$, and then extracted 3 times with 5 mL ether and pooled. The sample was air dried, resuspended in 0.3 mL ether, and 2 μL counted by liquid scintillation. A TLC plate (Kodak, Rochester, NY, USA) was loaded with 200,000 dpm, and TLC was run in an enclosed jar with toluene:acetic acid (9:1) for approximately 2.5 hr. The plate was exposed to X-ray film overnight. Comparison to laboratory standards (Aldrich Chemical Co., Milwaukee, WI, USA) confirmed conversion of lauric acid to 12-hydroxylauric acid and to DDDA from engineered *Pichia pastoris* strain SW64/65 (ATCC 74409). No conversion to DDDA from control *Pichia pastoris* was observed.

### EXAMPLE 2

Construction of *Candida maltosa* P450 Alk1-A Expression Cassette

The major alkane monooxygenase (P450Alk1-A) gene was isolated following PCR amplification and precisely fused to the *Candida maltosa* PGK promoter and terminator by PCR-mediated overlap extension. This technique allowed precise fusion of the PGK promoter and terminator to the translational start and stop codons, respectively, of the P450Alk1-A structural gene without any DNA sequence alterations that might alter PGK-mediated expression. The PGK promoter, comprising 766 bp of 5'-flanking DNA sequence upstream of the PGK structural gene (pos 56-756, not including primers), was amplified from ∼100 ng *Candida maltosa* ATCC 90677 [ade1, his5, ura3/ura3] genomic DNA using primers 7 (SEQ ID NO:7) and 8 (SEQ ID NO:8) to introduce a SpeI restriction site (indicated in lower case letters) necessary for subsequent subcloning and a 15 bp DNA sequence corresponding to the 5'-end of the P450Alk1-A gene (the indicated nucleotides are underlined):

Primer 7 - (SEQ ID NO:7):

5'-AactagtGGTAGAGCGATGGTTACATACGAC-3'

Primer 8 - (SEQ ID NO:8):

5'-TTGTTCTATAGCCATTCTAGTTAAGGCAATTGAT-3'

A 998 bp DNA fragment corresponding to the 5'-end of the P450Alk1-A gene (pos 47-977) was amplified from ∼20 ng pGEM-Alk1-A DNA, containing the *Candida maltosa* P450Alk1-A gene, using primers 9 (SEQ ID NO:9) and 10 (SEQ ID NO:10) to introduce a 15 bp DNA sequence corresponding to the 3'-end of the PGK promoter (the indicated nucleotides are underlined):

Primer 9 - (SEQ ID NO:9):

5'-GCCTTAACTAGAATGGCTATAGAACAAATTATTGAAGAA-3'

Primer 10 - (SEQ ID NO:10):

5'-TAAACCTGCAGTGGTATCTCTACCGGCA-3'

A 663 bp DNA fragment corresponding to the 3'-end of the P450Alk1-A gene (pos 1004-1596) was amplified from ∼20 ng pGEM-Alk1-A DNA using primers 11 (SEQ ID NO:11) and 12 (SEQ ID NO:12) to introduce a 15 bp DNA sequence corresponding to the 5'-end of the PGK terminator (the indicated nucleotides are underlined):

Primer 11 - (SEQ ID NO:11):

    5'-TGCCGGTAGAGATACCACTGCAGGTTTA-3'

Primer 12 - (SEQ ID NO:12):

    5'-<u>CATAAAAAATCAATT</u>CTATTTAGCAGAAATAAAAACACC-3'

The PGK terminator, comprising 588 bp of 3'-flanking DNA sequence downstream of the PGK structural gene (pos 2050-2571) was amplified from ~100 ng *Candida, maltosa* ATCC 90677 genomic DNA using the primers 13 (SEQ ID NO:13) and 14 (SEQ ID NO:14) to introduce a NheI restriction site (indicated in lower case letters) necessary for subsequent subcloning and a 15 bp DNA sequence corresponding to the 3'-end of the P450Alk1-A gene (the indicated nucleotides are underlined):

Primer 13 - (SEQ ID NO:13):

    5'-<u>ATTTCTGCTAAATAG</u>AATTGATTTTTTATGACACTTG-3'

Primer 14 - (SEQ ID NO:14):

    5'-AAAG<u>CTAGC</u>TTTGAAACAATCTGTGGTTG-3'

These PCRs were performed in a 50 µL volume using a Perkin Elmer Amplitaq kit. Amplification was carried out in a Perkin Elmer GeneAmp PCR System 9600 for 35 cycles, each comprising 1 min at 94 °C, 1 min at 50 °C and 2 min at 72 °C. Following the last cycle, there was a 5-min extension period at 72 °C, after which the samples were held at 4 °C prior to analysis by gel electrophoresis. The expected DNA fragments were isolated following preparative gel electrophoresis and purified using a Gene Clean kit (Bio101, Vista, CA).

The 766 bp DNA fragment comprising the PGK promoter and the 998 bp DNA fragment corresponding to the 5'-end of the P450Alk1-A gene were combined in a second PCR in which the complementary 3' end of the PGK promoter and the 5' end of the P450Alk1-A gene were annealed. Addition of the 5'-PGK and 3'-P450Alk1-A primers, primers 7 and 10, respectively, allowed amplification of a 1749 bp DNA fragment comprising a precise fusion of the PGK promoter to the 5' end of the P450Alk1-A gene. The 663 bp DNA fragment corresponding to the 3'-end of the P450ALK1A gene and the 588 bp DNA fragment comprising the PGK terminator and were combined in a second PCR in which the complementary 3' end of the P450Alk1-A gene and the 5' end of the PGK terminator were annealed. Addition of the 5'-P450Alk1-A and 3'-PGK primers, primers 11 and 14, respectively, allowed amplification of a 1236 bp DNA fragment comprising a precise fusion of the 3' end of the P450Alk1-A gene to the PGK terminator. These PCRs were performed in a 50 uL volume using a Perkin Elmer Amplitaq kit. Amplification was carried out in a Perkin Elmer GeneAmp PCR System 9600 for 35 cycles, each comprising 1 min at 94 °C, 1 min at 45 °C and 2 min at 72 °C. Following the last cycle, there was a 5-min extension period at 72 °C, after which the samples were held at 4 °C prior to analysis by gel electrophoresis. The expected DNA fragments were isolated following preparative gel electrophoresis and purified using a Gene Clean kit (Bio101).

The 1749 bp DNA fragment comprising a precise fusion of the PGK promoter to the 5' end of the P450Alk1-A gene was digested with SpeI and PstI and ligated to similarly digested pLitmus 38 (New England Biolabs, Beverly, MA). The ligated DNA was used to transform *E. coli* DH5α (GibcoBRL, Gaithersberg, MD) and analysis of the plasmid DNA from ampicillin-resistant transformants demonstrating white colony color in LB media (1% (w/v) tryptone; 1% (w/v) NaCl and 0.5% (w/v) yeast extract (Difco, Detroit, MI) containing X-gal (40 µg/mL) confirmed the presence of the expected plasmid, which was designated pLPA1. The 1236 bp DNA fragment comprising a precise fusion of the 3' end of the P450ALK1A gene to the PGK terminator was digested with PstI and NheI and ligated to similarly digested pLitmus 38. The ligated DNA was used to transform *E. coli* DH5α and analysis of the plasmid DNA from ampicillin-resistant transformants demonstrating white colony color in LB media containing X-gal confirmed the presence of the expected plasmid, which was designated pLA1T. Next, pLPA1 was linearized by digestion with PstI and NheI and ligated to the 1236 bp PstI/NheI DNA fragment from pLA1T. The ligated DNA was used to transform *E. coli* DH5α and analysis of the plasmid DNA from ampicillin-resistant transformants confirmed the presence of the expected plasmid, which was designated pLPA1T. Digestion of this plasmid with SpeI and NheI generates a 2985 bp expression cassette containing the Alk1-A gene precisely fused to the PGK promoter and terminator.

EXAMPLE 3

Construction of *Candida maltosa* P450 Alk3-A Expression Cassette

The major fatty acid monooxygenase (P450Alk3-A) gene was also isolated and precisely fused to the *Candida maltosa* PGK promoter and terminator by PCR-mediated overlap extension. The 766 bp PGK promoter (pos 56-756, not including primers) was amplified from ~100 ng *Candida maltosa* ATCC 90677 genomic DNA using primers 7 (SEQ ID NO:7) and 15 (SEQ ID NO:15) to introduce a SpeI restriction site (indicated in lower case letters) necessary for subsequent subcloning and a 15 bp DNA sequence corresponding to the 5'-end of the P450Alk3-A gene (the indicated nucleotides are underlined):

Primer 7 - (SEQ ID NO:7):
5'-AactagtGGTAGAGCGATGGTTACATACGAC-3'

Primer 15 - (SEQ ID NO:15):
5'-AAAGGAAACCGACATTCTAGTTAAGGCAATTGAT-3'

A 628 bp DNA fragment corresponding to the 5'-end of the P450Alk3-A gene (pos 62-655) was amplified from ~20 ng pGEM-Alk3-A DNA, containing the *Candida maltosa* P450Alk3-A gene, using primers 16 (SEQ ID NO:16) and 17 (SEQ ID NO:17) to introduce a 15 bp DNA sequence corresponding to the 3'-end of the PGK promoter (the indicated nucleotides are underlined):

Primer 16 - (SEQ ID NO:16):
5'-GCCTTAACTAGAATGTCGGTTTCCTTTGTTCACAACGTT-3'

Primer 17 - (SEQ ID NO:17):
5'-TCTTGGATATCGAAAGTTTTACCTTGAC-3'

A 1058 bp DNA fragment corresponding to the 3'-end of the P450Alk3-A gene (pos 652-1632) was amplified from ~20 ng pGEM-Alk3-A DNA using the primers 18 (SEQ ID NO:18) and 19 (SEQ ID NO:19) to introduce a 15 bp DNA sequence corresponding to the 5'-end of the PGK terminator (the indicated nucleotides are underlined):

Primer 18 - (SEQ ID NO:18):
5'-GTCAAGGTAAAACTTTCGATATCCAAGA-3'

Primer 19 - (SEQ ID NO:19):
5'-CATAAAAAATCAATTTTAGTACATTTGGATATTGGCACC-3'

The 588 bp PGK terminator (pos 2050-2571) was amplified from ~100 ng *Candida maltosa* ATCC 90677 genomic DNA using the primers 20 (SEQ ID NO:20) and 14 (SEQ ID NO:14) to introduce a NheI restriction site (indicated in lower case letters) necessary for subsequent subcloning and a 15 bp DNA sequence corresponding to the 3'-end of the P450Alk3-A gene (the indicated nucleotides are underlined):

Primer 20 - (SEQ ID NO:20):

5'-ATCCAAATGTACTAAAATTGATTTTTTATGACACTTG-3'

Primer 14 - (SEQ ID NO:14):
5'-AAAgctagcTTTGAAACAATCTGTGGTTG-3'

These PCRs were performed in a 50 uL volume using a Perkin Elmer Amplitaq kit. Amplification was carried out in a Perkin Elmer GeneAmp PCR System 9600 for 35 cycles, each comprising 1 min at 94 °C, 1 min at 50 °C and 2 min at 72 °C. Following the last cycle, there was a 5-min extension period at 72 °C, after which the samples were held at 4 °C prior to analysis by gel electrophoresis. The expected DNA fragments were purified using a Gene Clean kit (Bio101).

The 766 bp DNA fragment comprising the PGK promoter and the 628 bp DNA fragment corresponding to the 5'-end of the P450Alk3-A gene were combined in a second PCR in which the complementary 3' end of the PGK promoter and the 5' end of the P450Alk3-A gene were annealed. Addition of the 5'-PGK and 3'-P450Alk3-A primers, primers 7 and 17, respectively, allowed amplification of a 1379 bp DNA fragment comprising a precise fusion of the PGK promoter to the 5' end of the P450Alk3-A gene. The 1058 bp DNA fragment corresponding to the 3'-end of the P450Alk3-A gene and the 588 bp DNA fragment comprising the PGK terminator and were combined in a second PCR in which the complementary 3' end of the P450Alk3-A gene and the 5' end of the PGK terminator were annealed. Addition of the 5'-P450Alk3-A and 3'-PGK primers, primers 18 and 14, respectively, allowed amplification of a 1631 bp DNA fragment comprising a precise fusion of the 3' end of the P450Alk3-A gene to the PGK terminator. These PCRs were performed in a 50 μL volume using a Perkin Elmer Amplitaq kit. Amplification was carried out in a Perkin Elmer GeneAmp PCR System 9600 for 35 cycles, each comprising 1 min at 94 °C, 1 min at 45 °C and 2 min at 72 °C. Following the last cycle, there was a 5-min extension period at 72 °C, after which the samples were held at 4 °C prior to analysis by gel electrophoresis. The expected DNA fragments were isolated following preparative gel electrophoresis and purified using a Gene Clean kit (Bio101).

The 1379 bp DNA fragment comprising a precise fusion of the PGK promoter to the 5' end of the P450Alk3-A gene was digested with SpeI and EcoRV and ligated to similarly digested pLitmus 38. The ligated DNA was used to transform *E. coli* DH5α and analysis of the plasmid DNA from ampicillin-resistant transformants demonstrating white colony color in LB media containing X-gal confirmed the presence of the expected plasmid, which was designated pLPA3. The 1631 bp DNA fragment comprising a precise fusion of the 3' end of the P450Alk3-A gene to the PGK terminator was digested with EcoRV and NheI and ligated to similarly digested pLitmus 38. The ligated DNA was used to transform *E. coli* DH5α and analysis of the plasmid DNA from ampicillin-resistant transformants demonstrating white colony color in media containing X-gal confirmed the presence of the expected plasmid, which was designated pLA3T. Next, pLPA3 was linearized by digestion with EcoRV and NheI and was ligated to the 1631 bp EcoRV/NheI DNA fragment from pLA1T. The ligated DNA was used to transform *E. coli* DH5α and analysis of the plasmid DNA from ampicillin-resistant transformants confirmed the presence of the expected plasmid, which was designated pLPA3T. Digestion of this plasmid with SpeI and NheI generates a 3010 bp expression cassette containing the Alk3-A gene precisely fused to the PGK promoter and terminator.

EXAMPLE 4

Construction of *Candida maltosa* Cytochrome P450-NADPH Reductase Expression Cassette

The cytochrome P450-NADPH reductase (CPR) gene was also isolated and precisely fused to the *Candida maltosa* PGK promoter and terminator by PCR-mediated overlap extension. The 766 bp PGK promoter (pos 56-756, not including primers) was amplified from ~100 ng *Candida maltosa* ATCC 90677 genomic DNA using primers 7 (SEQ ID NO:7) and 21 (SEQ ID NO:21) to introduce a SpeI restriction site (indicated in lower case letters) necessary for subsequent subcloning and a 15 bp DNA sequence corresponding to the 5'-end of the CPR gene (the indicated nucleotides are underlined):

Primer 7 - (SEQ ID NO:7):

    5'-AactagtGGTAGAGCGATGGTTACATACGAC-3'

Primer 21 - (SEQ ID NO:21):

    5'-TTTATCTAATGCCATTCTAGTTAAGGCAATTGAT-3'

A 1038 bp DNA fragment corresponding to the 5'-end of the CPR gene (pos 88-1065) was amplified from ~20 ng pGEM-CPR DNA, containing the *Candida maltosa* CPR gene, using primers 22 (SEQ ID NO:22) and 23 (SEQ ID NO:23) to introduce a 15 bp DNA sequence corresponding to the 3'-end of the PGK promoter (the indicated nucleotides are underlined):

Primer 22 - (SEQ ID NO:22):

5'-<u>GCCTTAACTAGAATG</u>GCATTAGATAAATTAGATTT-3'

Primer 23 - (SEQ ID NO:23):

5'-AAGTGGAATCTAAAGCTTTTAATTCG-3'

A 1062 bp DNA fragment corresponding to the 3'-end of the CPR gene (pos 1090-2089) was amplified from ~20 ng pGEM-CPR DNA using primers 24 (SEQ ID NO:24) and 25 (SEQ ID NO:25) to introduce a 15 bp DNA sequence corresponding to the 5'-end of the PGK terminator (the indicated nucleotides are underlined):

Primer 24 - (SEQ ID NO:24):

5'-CGAATTAAAAGCTTTAGATTCCACTT-3'

Primer 25 - (SEQ ID NO:25):

5'-<u>CATAAAAAATCAATT</u>CTACCAAACATCTTCTTGGTA-3'

The 588 bp PGK terminator (pos 2050-2571) was amplified from ~100 ng *Candida maltosa* ATCC 90677 genomic DNA using primers 26 (SEQ ID NO:26) and 14 (SEQ ID NO:14) to introduce a NheI restriction site (indicated in lower case letters) necessary for subsequent subcloning and a 15 bp DNA sequence corresponding to the 3'-end of the CPR gene (the indicated nucleotides are underlined):

Primer 26 - (SEQ ID NO:26):

5'-GAAGATGTTTGGTAGAATTGATTTTTTATGACACTTG-3'

Primer 14 - (SEQ ID NO:14):

5'-AAAgctagcTTTGAAACAATCTGTGGTTG-3'

PCRs were performed in a 50 μL volume using a Perkin Elmer Amplitaq® kit. Amplification was carried out in a Perkin Elmer GeneAmp® PCR System 9600 for 35 cycles, each comprising 1 min at 94 °C, 1 min at 50 °C and 2 min at 72 °C. Following the last cycle, there was a 5-min extension period at 72 °C, after which the samples were held at 4 °C prior to analysis by gel electrophoresis. The expected DNA fragments were purified using a Gene Clean kit (Bio101).
The 766 bp DNA fragment comprising the PGK promoter and the 1038 bp DNA fragment corresponding to the 5'-end of the CPR gene were combined in a second PCR in which the complementary 3' end of the PGK promoter and the 5' end of the CPR gene were annealed. Addition of the 5'-PGK and 3'-CPR primers, primers 7 and 23, respectively, allowed amplification of a 1789 bp DNA fragment comprising a precise fusion of the PGK promoter to the 5' end of the CPR gene. The 1062 bp DNA fragment corresponding to the 3'-end of the CPR gene and the 588 bp DNA fragment comprising the PGK terminator and were combined in a second PCR in which the complementary 3' end of the CPR gene and the 5' end of the PGK terminator were annealed. Addition of the 5'-CPR and 3'-PGK primers, primers 24 and 14, respectively, allowed amplification of a 1635 bp DNA fragment comprising a precise fusion of the 3' end of the CPR gene to the PGK terminator. PCRs were performed in a 50 μL volume using a Perkin Elmer Amplitaq® kit. Amplification was carried out in a Perkin Elmer GeneAmp® PCR System 9600 for 35 cycles, each comprising 1 min at 94 °C, 1 min at 45 °C and 2 min at 72 °C. Following the last cycle, there was a 5-min extension period at 72 °C, after which the samples were held at 4 °C prior to analysis by gel electrophoresis. The expected DNA fragments were isolated following preparative gel electrophoresis and purified using a Gene Clean kit (Bio101).
The 1789 bp DNA fragment comprising a precise fusion of the PGK promoter to the 5' end of the CPR gene was digested with SpeI and HindIII and ligated to similarly digested pLitmus 38. The ligated DNA was used to transform *E. coli* DH5α and analysis of the plasmid DNA from ampicillin-resistant transformants demonstrating white colony color in LB media containing X-gal confirmed the presence of the expected plasmid, which was designated pLPR. The 1635 bp DNA fragment comprising a precise fusion of the 3' end of the CPR gene to the PGK terminator was digested with HindIII and NheI and ligated to similarly digested pLitmus 38. The ligated DNA was used to transform *E. coli* DH5α and analysis of the plasmid DNA from ampicillin-resistant transformants demonstrating white colony color in LB media containing X-gal confirmed the presence of the expected plasmid, which was designated pLRT. Next, pLPR was linearized by digestion with HindIII and NheI and was ligated to the 1635 bp HindIII/NheI DNA fragment from pLRT. The ligated DNA was used to transform *E. coli* DH5α and analysis of the plasmid DNA from ampicillin-resistant transformants confirmed the presence of the expected plasmid, which was designated pLPRT. Digestion of this plasmid with SpeI and NheI generates a 3424 bp expression cassette containing the CPR gene precisely fused to the PGK promoter and terminator.

EXAMPLE 5

Construction of *Candida maltosa* Strain Expressing Enhanced Alkane Hydroxylating Activity

Cytochrome P450 reductase is PCR-amplified from *Candida maltosa* ATCC 28140 using two primer sets. One set incorporates a BamHI site at the 5' end and a PstI site at the 3' end and amplifies a DNA fragment extending 2616 bases from a site 340 bases upstream of the reductase start codon. The other set incorporates a PstI site at the 5' and a SphI site at the 3' end with an XhoI site immediately upstream of the 3' SphI site and amplifies the same DNA fragment. The first DNA fragment containing the P450 reductase gene is then cloned between the BamHI and PstI sites in the pUC18 cloning vector (GibcoBRL, Baltimore, MD, USA) resulting in plasmid pRDF1. The later DNA fragment containing the P450 reductase gene is subcloned between the Pst1 and SphI sites resulting in plasmid RDF2. The *ade1* gene is then amplified from *Candida maltosa* ATCC 28140 using primers that incorporate an XhoI site at the 5' end and a SphI site at the 3' end. These primers amplify a DNA fragment extending 1396 bases from a site 284 bases upstream of the phosphoribosylamidoimidazole-succinocarboxamide synthetase start codon. The XhoI-*ade1*-SphI DNA fragment is cloned into plasmid pRDF2 between the Xho1 site and the Sph1 sites resulting in plasmid pRDF3. The same DNA segment containing the *ade1* gene is then again amplified from *Candida maltosa* ATCC 28140 using primers that incorporate a SmaI site at the 5' end and a BamHI site at the 3' end. This DNA fragment is cloned into pRDF3 between the SmaI and BamHI sites resulting in plasmid pRDF4.

A second plasmid construct is made by first amplifying the URA3 gene by PCR from *Candida maltosa* ATCC 28140 using primers that incorporate a BamHI site at the 5' end and a XbaI site at the 3' end. These primers amplify a DNA fragment extending 1171 bases from a site 285 bases upstream of the orotidine-5'-phosphate decarboxylase start codon. In addition, another set of primers that incorporate a SphI site at the 5' end and a HindIII site at the 3' end is used to amplify the same DNA fragment containing the URA3 gene. The BamHI - URA3 gene - XbaI fragment is cloned into the pUC18 cloning vector (GibcoBRL, Baltimore, MD, USA) between the BamHI and XbaI sites resulting in pRDF5. The later DNA fragment containing the URA3 gene is subcloned between the SphI and HindIII sites in pRDF5 resulting in pRDF6. The Alk1-A gene is then amplified from *Candida maltosa* ATCC 28140 using primers that incorporate a SalI site at the 5' end and a SphI site at the 3' end of the DNA fragment. These primers amplify a DNA fragment extending 1958 bases from a site 291 bases upstream of the P450Alk1-A start codon. The SalI - P450Alk1-A - SphI fragment is cloned into plasmid pRDF6 between the SalI site and the Sph1 sites resulting in plasmid pRDF7. The Alk3-A gene is then amplified from *Candida maltosa* ATCC 28140 using primers that incorporate a XbaI site at the 5' end and a SaiI site at the 3' end. These primers amplify a DNA fragment extending 2063 bases from a site 276 bases upstream of the P450Alk3-A start codon. This DNA fragment is cloned into pRDF6 between the XbaI and SalI sites resulting in plasmid pRDF8.

Plasmid RDF4 is used to transform *Candida maltosa* ATCC 90677 [ade1, his5] to adenine prototrophy by spheroplast method (Cregg et al., *Mol. Cell Biol.,* 5:3376-3385, (1985)) resulting in plasmid integration into the genome. High copy number transformants are selected by screening of transformants using the Southern blot method. Clones yielding the strongest signal contain the highest number of integrated copies of the P450 reductase gene. A high copy number transformant is retransformed to uracil prototrophy with plasmid RDF8 resulting in plasmid integration into the genome. Transformants are selected for growth on dodecane in the presence of increasing amounts of 1-dodecyne. Clones expressing the highest levels of P450 activity are able to grow at the highest 1-dodecyne concentrations. In addition, PCR and/or Southern blot analysis is used to verify the integration of expression cassettes for Alk1-A, Alk3-A and P450 reductase genes into the genome of a double transformant.

Double-transformed *Candida maltosa* ATCC 90677 strains are grown to late log phase (Å 48 h) in YEP (10 g/L yeast extract + 20 g/L peptone, pH 8) + 0.05% Tween 80 + 10 g/L dodecane at 30 °C with shaking at 250 rpm. Cells are centrifuged and washed once in 10% YEP, pH 8. Cells are resuspended in 10% YEP, pH 8 + 0.05% Tween 80 + 10 g/L dodecane at 30 °C and shaken at 250 rpm for 24 h. The loss of dodecane and the production of dodecanedioic acid are measured followed extraction by GC analysis. The production of dodecanedioic acid is found to be enhanced in the doubly-transformed strains as compared to the original ATCC 90677 strain.

EXAMPLE 6

Construction of a POX4 Disruption Cassette

Gene specific primers were used to amplify the *Candida maltosa* POX4 gene from genomic DNA, while adding unique restriction sites to their flanking regions for subsequent ligation into plasmids. A 1567 bp *Candida maltosa* POX4 gene fragment (pos 908-2412) was PCR-amplified from ~100 ng *Candida maltosa* ATCC 90677 [ade1, his5, ura3/ura3] genomic DNA in 50 µL of a standard PCR mix using a Perkin Elmer Amplitaq kit and primers 27 (SEQ ID NO:27) and 28 (SEQ ID NO:28) to introduce the BamHI cleavage sites (indicated in lower case letters) necessary for subsequent subcloning:

> Primer 27 - (SEQ ID NO:27):
>
>    5'-GGGTCACggatccAATGTTGCTGG-3'
>
> Primer 28 - (SEQ ID NO:28):
>
>    5'-GCAGCAGTGTATggatccTTAGTGTTCTTTGGTGGG-3'

Amplification was carried out in a Perkin Elmer GeneAmp PCR System 9600 for 35 cycles, each comprising 1 min at 94 °C, 1 min at 55 °C and 2 min at 72 °C. Following the last cycle, there was a 5-min extension period at 72 °C, after which the samples were held at 4 °C prior to analysis by gel electrophoresis. The reactions containing the expected 1567 bp DNA fragment were extracted with phenol:chloroform:isoamyl alcohol (25:24:1 v/v), and the DNA was precipitated with ethanol and resuspended in TE buffer (10 mM Tris pH 7.5, 1 mM EDTA). The 1567 bp DNA fragment was digested with BamHI and ligated to BamHI-digested pBR322 (New England Biolabs, Beverly, MA). The ligated DNA was used to transform *E. coli* DM1 (GibcoBRL, Gaithersberg, MD) and analysis of the plasmid DNA from ampicillin-resistant, tetracycline-sensitive transformants confirmed the presence of the expected plasmid, which was designated pBR-CMPOX4.

A 1184 bp DNA fragment containing the *Candida maltosa* URA3 gene (pos 8-1192) was PCR-amplified from ~100 ng *Candida maltosa* ATCC 90625 [ade1, his5, ura3/ura3] genomic DNA in 50 µL of a standard PCR mixture using a Perkin Elmer Amplitaq kit and primers 29 (SEQ ID NO:29) and 30 (SEQ ID NO:30) to introduce the BclI cleavage sites (indicated in lower case letters) necessary for subsequent subcloning:

> Primer 29 - (SEQ ID NO:29):
>
>    5'-GACTTtgatcaATTTTGGTACCAT-3'
>
> Primer 30 - (SEQ ID NO:30):
>
>    5'-AGGGTACCATGAAGTTTTAGACTCTtgatcaCT-3'

Amplification was carried out in a Perkin Elmer GeneAmp PCR System 9600 for 35 cycles, each comprising 1 min at 94 °C, 1 min at 50 °C and 2 min at 72 °C. Following the last cycle, there was a 5-min extension period at 72 °C, after which the samples were held at 4 °C prior to analysis by gel electrophoresis. The reactions containing the expected 1184 bp DNA fragment were extracted with phenol:chloroform:isoamyl alcohol (25:24:1 v/v), and the DNA was precipitated with ethanol and resuspended in TE buffer (10 mM Tris pH 7.5, 1 mM EDTA).

The 1184 bp PCR fragment containing the URA3 selectable marker was digested with BclI and ligated to pBR-CMPOX4 which had been digested with BclII and treated with calf intestinal phosphatase. The ligated DNA was used to transform *E. coli* DH5α competant cells (GibcoBRL, Gaithersberg, MD) and analysis of the plasmid DNA from ampicillin-resistant confirmed the presence of the expected plasmid, which was designated pBR-pox4::URA3. Digestion of this plasmid with BamHI released a 2.8 kb linear POX4 disruption cassette containing the URA3 selectable marker flanked by 770 bp of 5'- and 734 bp of 3'-homology to the POX4 target gene.

EXAMPLE 7

Construction of a *Candida maltosa* Strain with Disrupted POX4 Genes

A β-oxidation-blocked strain of *Candida maltosa* was developed by sequential disruption of both POX4 genes encoding acyl-CoA oxidase, which catalyzes the first reaction in the pathway. *Candida maltosa* ATCC 90677 lacks the URA3 gene product, orotidine-5'-monophosphate decarboxylase, and requires uracil for growth. The 2.8 kb linear POX4 disruption cassette derived from plasmid pBR-pox4:URA3 was used to transform *Candida maltosa* ATCC 90677 to uracil prototrophy as described by Gietz and Woods in Molecular Genetics of Yeast: A Practical Approach (Johnson, J.R., ed.) pp. 121-134, Oxford University Press (1994). Ura+ transformants were selected in a supplemented minimal media containing 0.67 g/L Yeast Nitrogen Base (Difco, Detroit, MI), 2% (w/v) glucose, 2% Bacto-agar (Difco) and 20 mg/L each of adenine sulfate and L-histidine.

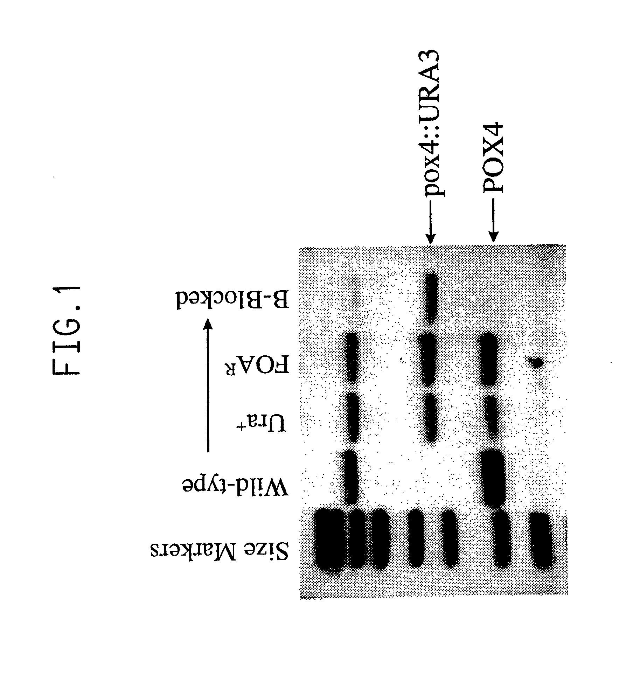

Southern hybridization of XmnI-digested genomic DNA from 20 independent Ura+ transformants to a POX4 probe showed each to contain the expected disruption of a single copy of POX4 (see Figure 1, lane marked Ura+). These results indicate that the ends of linear DNA are highly recombinagenic and dictate the precise site of integration into the *Candida maltosa* genome. Since *Candida maltosa* is a diploid yeast, these transformants also contained a second functional copy of the POX4 gene that had to be disrupted in order to inactivate the β-oxidation pathway. To sequentially disrupt both copies of the POX4 gene in a single strain, uracil-requiring revertants were first counter-selected in supplemented minimal media also containing 2 mg/mL 5-fluoroorotic acid (5-FOA), a toxic analogue of a uracil biosynthesis pathway intermediate that is incorporated only into Ura+ cells. Thus, only Ura- cells survive and grow in the combined presence of 5-FOA and uracil. Several FOA-resistant, uracil-requiring derivatives were isolated and one which retained the original POX4 disruption and also showed low reversion frequency to uracil prototrophy (See Figure 1, lane marked FOA^R) was transformed a second time to uracil prototrophy using the same pox4::URA3 disruption cassette derived from plasmid pBR-pox4:URA3. Following transformation, about half of the resulting Ura+ transformants were unable to grow on dodecane as the sole carbon source, suggesting that their β-oxidation pathway had been blocked. Analysis of these transformants by Southern hybridization confirmed that both genomic copies of the POX4 gene were disrupted (See Figure 1, lane marked β-blocked). Subsequent analyses have confirmed the absence of any remaining acyl-CoA oxidase activity in these transformants and their ability to convert dodecane to DDDA. Each URA3-mediated gene disruption conveniently provides a distinct integration target for subsequent amplification of the cytochrome P450 monooxygenase and cytochrome P450-NADPH reductase genes.

EXAMPLE 8

Construction of *Candida maltosa* Strain with Disrupted POX4 Genes and with Other Auxotrophic Markers Removed

The *Candida* maltosa ADE1 gene was isolated by PCR, using primers 31 (SEQ ID NO:31) and 32 (SEQ ID NO:32), which incorporate NdeI sites (indicated in lower case letters), and subcloned into the NdeI site of pUC18m (a derivative of pUC18, in which SpeI and NheI restriction sites have been inserted between SaiI and XbaI in the polylinker region), to generate pSW81:

Primer 31 - (SEQ ID NO:31):
5'-CTTCTTCAAACCTTcatatgACATTGTTTCG-3'

Primer 32 - (SEQ ID NO:32):
5'-CTAATGGTCAAGcatatgTTGCATTATC-3'

The *Candida maltosa* HIS5 gene was isolated by PCR, using primers 33 (SEQ ID NO:33) and 34 (SEQ ID NO:34), which incorporate NdeI sites (indicated in lower case letters), and subcloned into the NdeI site of pUC18m, to generate pSW82:

Primer 33 - (SEQ ID NO:33):
5'-TTTGGTTGACTcatatgTGAGCGCGGTAAAG-3'

Primer 34 - (SEQ ID NO:34):
5'-GTTTTGTCTGGCcatatgTTGAACTGGATGG-3'

One β-blocked *Candida maltosa* transformant described in Example 7 (designated *Candida maltosa* 11-11) was further modified to eliminate the two remaining auxotrophic requirements, adenine and histidine, which derive from ATCC 90677. This removal was accomplished by co-transforming *Candida maltosa* 11-11 with pSW81 and pSW82, by lithium chloride transformation method essentially as described (Gietz et al., *Methods Mol. Cell. Biol.,* 5:255-269, (1996)), and selecting at 30 °C on minimal plates (1.34% Yeast Nitrogen Base without amino acids, 2% (w/v) glucose) without adenine or histidine supplements. The resulting strain is designated *Candida maltosa* SW81/82, and is identified by ATCC Accession No. 74431.

EXAMPLE 9

Construction of *Candida maltosa* Strain Expressing Enhanced Alkane Hydroxylating Activity and Disrupted POX4 Genes

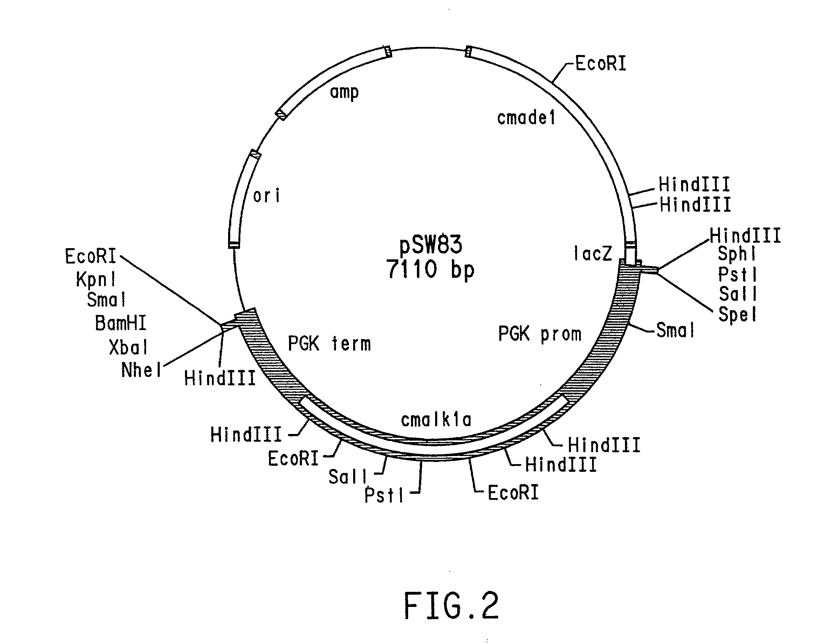

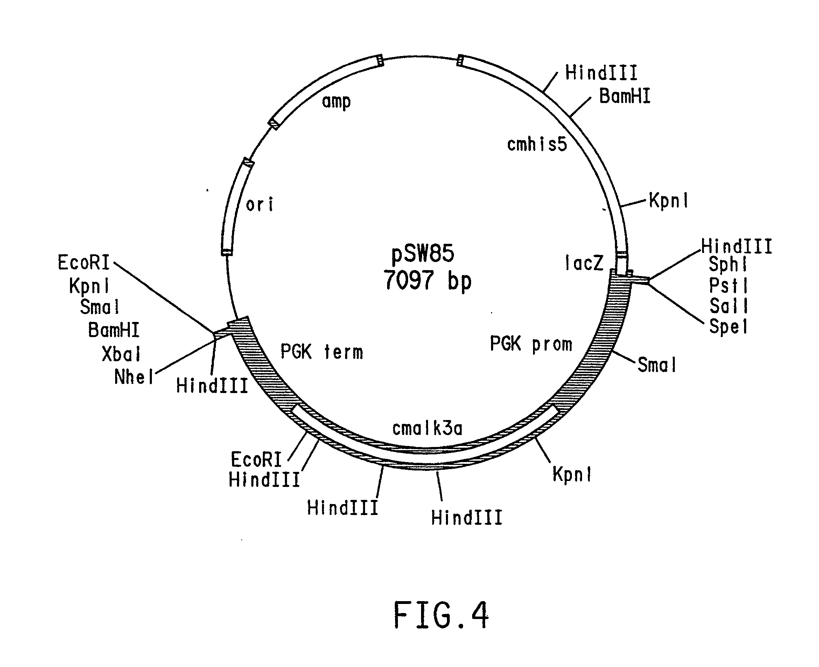

The *Candida maltosa* cytochrome P450 Alk1-A expression cassette, as described in Example 2, was subcloned into pSW81 (as described in Example 8) between SpeI and NheI, to generate pSW83 (see Figure 2). The *Candida maltosa* cytochrome P450-NADPH reductase expression cassette, as described in Example 4, was subcloned into pSW83 NheI, to generate pSW84, which contains expression cassettes for both cytochrome P450Alk1-A and cytochrome P450-NADPH reductase, plus the adel selectable marker (see Figure 4). The *Candida maltosa* cytochrome P450Alk3-A expression cassette, as described in Example 3, was subcloned into pSW82 between SpeI and Nhe1, to generate pSW85. The *Candida maltosa* cytochrome P450-NADPH reductase-expression cassette, as described in Example 4, was subcloned into pSW85 Nhe1, to generate pSW87, which contains expression cassettes for both cytochrome P450Alk3-A and cytochrome P450-NADPH reductase, plus the his5 selectable marker.

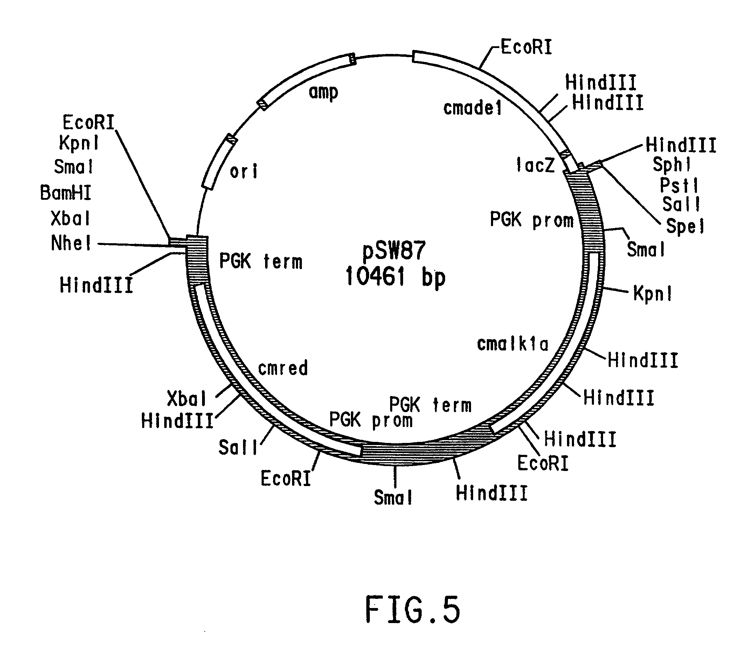

The *Candida maltosa* β-blocked strain designated 11-11 (as described in Example 7) was co-transformed with pSW84 (see Figure 4) and pSW87 (see Figure 5), by lithium chloride transformation method essentially as described (Gietz et al., *Methods Mol. Cell. Biol.,* 5:255-269, (1996)), and selected at 30 °C on minimal plates (1.34% Yeast Nitrogen Base without amino acids, 2% (w/v) glucose) supplemented with adenine, or supplemented with histidine, or without supplements. PCR and/or Southern analyses confirmed chromosomal integration of expression cassettes for cytochrome P450Alk1-A and cytochrome P450-NADPH reductase (strain designated *Candida maltosa* SW84), or cytochrome P450Alk3-A and cytochrome P450-NADPH reductase (strain designated *Candida maltosa* SW87), or cytochrome P450Alk1-A, cytochrome P450Alk3-A, and cytochrome P450-NADPH reductase (strain designated *Candida maltosa* SW84/87). One *Candida maltosa* SW84/87 double transformant, designated *Candida maltosa* SW84/87.2 is identified by ATCC Accession No. 74430. After growing *Candida maltosa* SW84, *Candida maltosa* SW87, and *Candida maltosa* SW84/87 at 30 °C in YEPD (1% yeast extract, 2% peptone, 2% glucose) to saturation (24 h), cells were harvested by centrifugation, broken with glass beads to produce a semi-clear lysate as described in Example 1, and assayed for hydroxylation activity as described in Example 1. *Candida maltosa* SW84, *Candida maltosa* SW87 and *Candida maltosa* 84/87 each demonstrate conversion of lauric acid to DDDA.

EXAMPLE 10

Production of Dodecanedioic Acid (DDDA) from Dodecane by *Candida maltosa* Strain SW81/82 (ATCC 74431)

A 5 mL seed inoculum of *Candida maltosa* SW81/82 (ATCC 74431) was grown for 24 h at 30 °C with shaking at 250 rpm in YEPD medium (10 g/L yeast extract, 20 g/L peptone and 20 g/L glucose). The resulting mixture was inoculated into 350 mL of pH 5 yeast minimal medium (3 g/L $(NH_4)_2SO_4$, 6.6 g/L $KH_2PO_4$, 0.4 g/L $K_2HPO_4$, 0.6 g/L anhydrous $MgSO_4$, 4 g/L yeast extract, 75 g/L glucose, 100 µg/L biotin, 13 mg/L $FeSO_4 \cdot 7H_2O$, 2 mg/L $CuSO_4 \cdot 5H_2O$, 20 mg/L $ZnSO_4 \cdot 7H_2O$, 6 mg/L $MnSO_4 \cdot H_2O$, 2 mg/L $Co(NO_3)_2 \cdot 6H_2O$, 3 mg/L $NaMoO_4 \cdot 2H_2O$ and 1.6 mg/L KI) and grown for 24 h at 30 °C with shaking at 250 rpm. A fermenter (Braun) containing 7 L of pH 5 yeast minimal medium was inoculated with the overnight culture. The fermenter was maintained at minimal airflow and agitation until dissolved oxygen dropped to 20% of atmospheric. The dissolved oxygen was then raised to approximately 80% of atmospheric and maintained through fermenter control of aeration up to 2 vvm and agitation up to 1400 rpm at 30 °C. The addition of 10% w/v $NH_4OH$ provided nitrogen for cell growth and also maintained the medium at pH 5. After approximately 14 h, glucose concentration dropped to near zero. Dodecane was then added to a final concentration of approximately 20 g/L. The pH of the medium was adjusted to 7.5 through the addition of 20% w/v KOH. Further additions of 20% w/v KOH to the medium maintained the pH at 7.5 for the remainder of the fermentation. Dodecane concentrations were monitored periodically and maintained above 3 g/L. In addition, glucose was fed at a slow rate in the range of 0.2 to 0.8 g glucose/min and glucose concentration was monitored. The slow rate of glucose feed was used to maintain the glucose concentration below 1 g glucose/L. Approximately 69 h after dodecane addition, material from the fermenter was harvested and analyzed for DDDA.

DDDA was recovered from the whole fermenter liquor (cells and supernatant) by acidifying the liquor to pH 2 with 2M phosphoric acid and extracting the precipitated material into 3 x 5 mL methyl-tertiary butyl ether. A portion of the ether extract was evaporated to dryness and the recovered DDDA was reacted with MSTFA (N-methyl-N-trimethylsilyltrifluoroacetamide) to form a derivative detectable by GC under the standard conditions specified above.

DDDA was present at 28.8 g/L or a total yield of 187 g from the fermenter. The mean production rate is 2.7 g DDDA/h.

EXAMPLE 11

Production of Dodecanedioic Acid (DDDA) from Dodecane by *Candida maltosa* 84/87.2 (ATCC 74430

A 10 mL seed inoculum of *Candida maltosa* strain 84/87.2 (ATCC 74430) was grown for 24 h at 30 °C with shaking at 250 rpm in YEPD medium (10 g/L yeast extract, 20 g/L peptone and 20 g/L glucose). The resulting mixture was inoculated into 2 x 350 mL of pH 5 yeast minimal medium (3 g/L $(NH_4)_2SO_4$, 6.6 g/L $KH_2PO_4$, 0.4 g/L $K_2HPO_4$, 0.6 g/L anhydrous $MgSO_4$, 4 g/L yeast extract, 75 g/L glucose, 100 µg/L biotin, 13 mg/L $FeSO_4·7H_2O$, 2 mg/L $CuSO_4·5H_2O$, 20 mg/L $ZnSO_4·7H_2O$, 6 mg/L $MnSO_4·H_2O$, 2 mg/L $Co(NO_3)_2·6H_2O$, 3 mg/L $NaMoO_4·2H_2O$ and 1.6 mg/L KI) and grown for 24 h at 30 °C with shaking at 250 rpm. A fermenter (Braun) containing 7 L of pH 5 yeast minimal medium was inoculated with 525 mL of overnight culture. The fermenter was maintained at minimal airflow and agitation until dissolved oxygen dropped to 20% of atmospheric. The dissolved oxygen was then raised to approximately 80% of atmospheric and maintained through fermenter control of aeration up to 2 vvm and agitation up to 1400 rpm at 30 °C. The addition of 10% w/v $NH_4OH$ provided nitrogen for cell growth and also maintained the pH of the medium at 5. After approximately 18 h, glucose concentration dropped to near zero. Dodecane was then added to a final concentration of approximately 20 g/L. The pH of the medium was adjusted to 7.5 through the addition of 20% w/v KOH. Further addition of 20% w/v KOH maintained pH of the medium at 7.5 for the remainder of the fermentation. Dodecane concentrations were monitored periodically and maintained above 3 g/L. In addition, glucose was fed at a slow rate in the range of 0.2 to 0.8 g glucose/min and glucose concentration was monitored. The slow rate of glucose feed was used to maintain the glucose concentration below 1 g glucose/L. Approximately 51 h after dodecane addition, material from the fermenter was harvested and analyzed for DDDA.

DDDA was recovered from the whole fermenter liquor (cells and supernatant) by acidifying the liquor to pH 2 with 2M phosphoric acid and extracting the precipitated material into 3 x 5 mL methyl-tertiary butyl ether. A portion of the ether extract was evaporated to dryness and the recovered DDDA was reacted with MSTFA (N-methyl-N-trimethylsilyltrifluoroacetamide) +1% TMCS (trimethylchlorosilane) to form a derivative detectable by GC under standard conditions specified above.

DDDA was present at 21.6 g/L or a total yield of 173 g from the fermenter. The mean production rate for *Candida maltosa* SW84/87.2 was 3.4 g DDDA/h, a 20% improvement over the production rate for *Candida maltosa* SW81/82.

EXAMPLE 12

Production of Dodecanedioic Acid (DDDA) from Lauric Acid Methyl Ester by *Candida maltosa* Strain 84/87-2 (ATCC 77430)

A 10 mL seed inoculum of strain 84/87-2 (ATCC 77430) is grown for 24 h at 30 °C and 250 rpm in YEPD medium (10 g/L yeast extract, 20 g/L peptone and 20 g/L glucose). This seed is inoculated into 2 x 350 mL of pH 5 yeast minimal medium (3 g/L $(NH_4)_2SO_4$, 6.6 g/L $KH_2PO_4$, 0.4 g/L $K_2HPO_4$, 0.6 g/L anhydrous $MgSO_4$, 4 g/L yeast extract, 75 g/L glucose, 100 µg/L biotin, 13 mg/L $FeSO_4·7H_2O$, 2 mg/L $CuSO_4·5H_2O$, 20 mg/L Zn $SO_4·7 H_2O$, 6 mg/L $MnSO_4·H_2O$, 2 mg/L $Co(NO_3)_2·6 H_2O$, 3 mg/L $NaMoO_4·2 H_2O$ and 1.6 mg/L KI) and grown for 24 h at 30 °C and 250 rpm. A fermenter (Braun) containing 7 L of pH 5 yeast minimal medium is inoculated with 525 mL of the overnight culture. The fermenter is maintained at minimal airflow and agitation until dissolved oxygen drops to 20% of atmospheric. The dissolved oxygen is then raised to approximately 80% of atmospheric and maintained through fermenter control of aeration up to 2 vvm and agitation up to 1400 rpm at 30 °C. The addition of 10% w/v $NH_4OH$ provides nitrogen for cell growth and also maintains pH of the medium at 5. When the glucose concentration in the fermenter drops to < 1 g/L, lauric acid methyl ester is added to a final concentration of approximately 5 g/L. The pH of the medium is adjusted to 7.5 through the addition of 20% w/v KOH. Further addition of 20% w/v KOH maintains pH 7.5 of the medium for the remainder of the fermentation. Lauric acid methyl ester concentrations are monitored periodically and the concentration is maintained above 3 g/L. In addition, glucose is fed at a slow rate in the range of 0.2 to 0.8 g glucose/min and glucose concentration is monitored. The slow rate of glucose feed is used to maintain the glucose concentration below 1 g glucose/L. After 48 h lauric acid methyl ester addition is stopped and the reaction allowed to procede until lauric acid methyl ester is no longer detectable. Material from the fermenter is harvested and DDDA is recovered.

EP 1 273 663 A2

INDICATIONS RELATING TO A DEPOSITED MICROORGANISM

(PCT Rule 13bis)

A. The indications made below relate to the microorganism referred to in the description
on page __10__ , line __5__ .

B. IDENTIFICATION OF DEPOSIT          Further deposits are identified on an additional sheet ☐

Name of depositary institution

AMERICAN TYPE CULTURE COLLECTION

Address of depositary institution (including postal code and country)

10801 University Blvd.
Manassas, Virginia 20110-2209
USA

| Date of deposit | Accession Number |
|---|---|
| 03 April 1997 (03.04.97) | 74409 |

C. ADDITIONAL INDICATIONS (leave blank if not applicable)    This information is continued on an additional sheet ☐

In respect of those designations in which a European patent is sought,
a sample of the deposited microorganism will be made available until
the publication of the mention of the grant of the European patent or
until the date on which the application has been refused or withdrawn
or is deemed to be withdrawn, only by the issue of such a sample to an
expert nominated by the person requesting the sample. (Rule 28(4) EPC)

D. DESIGNATED STATES FOR WHICH INDICATIONS ARE MADE (if the indications are not for all designated States)

E. SEPARATE FURNISHING OF INDICATIONS (leave blank if not applicable)

The indications listed below will be submitted to the International Bureau later (specify the general nature of the indications e.g., "Accession Number of Deposit")

| ━━ For receiving Office use only ━━ | ━━ For International Bureau use only ━━ |
|---|---|
| ☑ This sheet was received with the international application | ☐ This sheet was received by the International Bureau on: |
| Authorized officer<br><br>Melvin Brooks | Authorized officer |

Form PCT/RO/134 (July 1992)                    40

23

## INDICATIONS RELATING TO A DEPOSITED MICROORGANISM

(PCT Rule 13*bis*)

---

A. The indications made below relate to the microorganism referred to in the description

on page __10__ , line __5__

---

**B. IDENTIFICATION OF DEPOSIT**  Further deposits are identified on an additional sheet ☐

Name of depositary institution

AMERICAN TYPE CULTURE COLLECTION

---

Address of depositary institution *(including postal code and country)*

10801 University Blvd.
Manassas, Virginia 20110-2209
USA

---

| Date of deposit | Accession Number |
|---|---|
| 10 December 1997 (10.12.97) | 74431 |

---

**C. ADDITIONAL INDICATIONS** *(leave blank if not applicable)*  This information is continued on an additional sheet ☐

In respect of those designations in which a European patent is sought,
a sample of the deposited microorganism will be made available until
the publication of the mention of the grant of the European patent or
until the date on which the application has been refused or withdrawn
or is deemed to be withdrawn, only by the issue of such a sample to an
expert nominated by the person requesting the sample. (Rule 28(4) EPC)

---

**D. DESIGNATED STATES FOR WHICH INDICATIONS ARE MADE** *(if the indications are not for all designated States)*

---

**E. SEPARATE FURNISHING OF INDICATIONS** *(leave blank if not applicable)*

The indications listed below will be submitted to the International Bureau later *(specify the general nature of the indications e.g., "Accession Number of Deposit")*

---

| ── For receiving Office use only ── | ── For International Bureau use only ── |
|---|---|
| ☑ This sheet was received with the international application | ☐ This sheet was received by the International Bureau on: |
| Authorized officer  *Melvin Brooks* | Authorized officer |

Form PCT/RO/134 (July 1992)                41

## INDICATIONS RELATING TO A DEPOSITED MICROORGANISM

### (PCT Rule 13bis)

| A. The indications made below relate to the microorganism referred to in the description |
|---|
| on page __10__ , line __5__ |

| B. IDENTIFICATION OF DEPOSIT | Further deposits are identified on an additional sheet ☐ |
|---|---|

Name of depositary institution

AMERICAN TYPE CULTURE COLLECTION

Address of depositary institution *(including postal code and country)*

10801 University Blvd.
Manassas, Virginia 20110-2209
USA

| Date of deposit | Accession Number |
|---|---|
| 10 December 1997 (10.12.97) | 74430 |

**C. ADDITIONAL INDICATIONS** *(leave blank if not applicable)*     This information is continued on an additional sheet ☐

In respect of those designations in which a European patent is sought, a sample of the deposited microorganism will be made available until the publication of the mention of the grant of the European patent or until the date on which the application has been refused or withdrawn or is deemed to be withdrawn, only by the issue of such a sample to an expert nominated by the person requesting the sample. (Rule 28(4) EPC)

**D. DESIGNATED STATES FOR WHICH INDICATIONS ARE MADE** *(if the indications are not for all designated States)*

**E. SEPARATE FURNISHING OF INDICATIONS** *(leave blank if not applicable)*

The indications listed below will be submitted to the International Bureau later *(specify the general nature of the indications e.g., "Accession Number of Deposit")*

| ─── For receiving Office use only ─── | ─── For International Bureau use only ─── |
|---|---|
| ☑ This sheet was received with the international application | ☐ This sheet was received by the International Bureau on: |
| Authorized officer

*Melvin Brooks* | Authorized officer |

Form PCT/RO/134 (July 1992)                    42

**Claims**

1.  A method for the bioproduction of $C_6$ to $C_{22}$ mono- and di-carboxylic acids comprising

    a) contacting, under aerobic conditions, a transformed *Pichia pastoris* **characterized by** a genetically-engineered alkane hydroxylating activity with at least one $C_6$ to $C_{22}$ straight chain hydrocarbon; and
    b) recovering the $C_6$ to $C_{22}$ mono- and di-carboxylic acids.

2.  The method of Claim 1, wherein the at least one $C_6$ to $C_{22}$ straight chain hydrocarbon is selected from the group consisting of hexane, heptane, octane, nonane, decane, undecane, dodecane, tridecane, tetradecane, pentadecane, hexadecane, heptadecane, octadecane, nonadecane, eicosane, reneicosane, docosane and their respective mono-carboxylic acids and esters.

3.  The method of Claim 1, wherein the at least one $C_6$ to $C_{22}$ straight chain hydrocarbon is dodecane; and the product recovered is dodecanedioic acid.

4.  The method of any one of Claims 1 to 3, wherein the transformed *Pichia pastoris* is strain SW 64/65 identified as ATCC 74409.

5.  A transformed *Pichia pastoris* comprising

    a) at least one copy of a foreign gene encoding cytochrome P450 monooxygenase; and, optionally,
    b) at least one copy of a foreign gene encoding cytochrome P450 reductase,

    each gene being operably linked to suitable regulatory elements such that alkane hydroxylating activity is enhanced upon contact with at least one $C_6$ to $C_{22}$ straight chain hydrocarbon.

**6.** The transformed *Pichia pastoris* of Claim 5, wherein the foreign gene encoding cytochrome P450 monooxygenase is selected from the group consisting of Alk1-A (D12475), Alk2-A (X55881), Alk3-A (X55881), Alk4-A (D12716), Alk5-A (D12717), Alk6-A (D12718), Alk7 (D12719), and Alk8 (D12719).

**7.** The transformed *Pichia pastoris* of Claim 5, wherein the foreign gene encoding cytochrome P450 reductase is cytochrome P450 reductase (D25327).

**8.** A transformed *Pichia pastoris* strain **characterized by** an enhanced alkane hydroxylating activity and comprising,

a) at least one DNA fragment from *Candida maltosa* ATCC 90677 selected from the group of DNA fragments encoding cytochrome P450 monooxygenase A1k1-A and cytochrome P450 monooxygenase Alk3-A; and, optionally,
b) at least one DNA fragment from *Candida maltosa* ATCC 90677 encoding cytochrome P450 reductase,

each DNA fragment being operably linked to suitable regulatory elements such that alkane hydroxylating activity is enhanced upon contact with at least one $C_6$ to $C_{22}$ straight chain hydrocarbon.

**9.** A transformed *Pichia pastoris* strain SW64/65 identified as ATCC 74409.

FIG.1

FIG.2

EP 1 273 663 A2

FIG.3

FIG. 4

FIG.5

EP 1 273 663 A2

SEQUENCE LISTING

<110>   E. I. DU PONT DE NEMOURS AND COMPANY

<120>   TRANSFORMED YEAST STRAINS AND THEIR USE FOR THE PRODUCTION
        OF MONOTERMINAL AND DITERMINAL ALIPHATIC CARBOXYLATES

<130>   CL-1035-A

<140>
<141>

<150>   US 60/053,215
<151>   21 JULY 1997

<160>   34

<170>   PatentIn Ver. 2.0

<210>   1
<211>   26
<212>   DNA
<213>   Sense primer

<400>   1
aggatccatg gcattagata aattag                                    26

<210>   2
<211>   25
<212>   DNA
<213>   Antisense primer

<400>   2
acctaggcta ccaaacatct tcttg                                     25

<210>   3
<211>   26
<212>   DNA
<213>   Sense primer

<400>   3
cggtaccatg gctatagaac aaatta                                    26

<210>   4
<211>   25
<212>   DNA
<213>   Antisense primer

<400>   4
agggcccttt agcagaaata aacac                                     25

<210>   5
<211>   26
<212>   DNA
<213>   Sense primer

<400>   5
actcgagatg ccggtttcct ttgttc                                    26

<210>   6
<211>   24
<212>   DNA
<213>   Antisense primer

<400>   6
agggcccgta catttggata ttgg                                      24

```
<210>   7
<211>   31
<212>   DNA
<213>   Sense primer

<400>   7
aactagtggt agagcgatgg ttacatacga c                              31

<210>   8
<211>   34
<212>   DNA
<213>   Antisense primer

<400>   8
ttgttctata gccattctag ttaaggcaat tgat                           34

<210>   9
<211>   39
<212>   DNA
<213>   Sense primer

<400>   9
gccttaacta gaatggctat agaacaaatt attgaagaa                      39

<210>   10
<211>   28
<212>   DNA
<213>   Antisense primer

<400>   10
taaacctgca gtggtatctc taccggca                                  28

<210>   11
<211>   28
<212>   DNA
<213>   Sense primer

<400>   11
tgccggtaga gataccactg caggttta                                  28

<210>   12
<211>   39
<212>   DNA
<213>   Antisense primer

<400>   12
cataaaaaat caattctatt tagcagaaat aaaaacacc                      39

<210>   13
<211>   37
<212>   DNA
<213>   Sense primer

<400>   13
atttctgcta aatagaattg attttttatg acacttg                        37

<210>   14
<211>   29
<212>   DNA
<213>   Antisense primer

<400>   14
aaagctagct ttgaaacaat ctgtggttg                                 29
```

27

```
<210>   15
<211>   34
<212>   DNA
<213>   Antisense primer

<400>   15
aaaggaaacc gacattctag ttaaggcaat tgat                    34

<210>   16
<211>   39
<212>   DNA
<213>   Sense primer

<400>   16
gccttaacta gaatgtcggt ttcctttgtt cacaacgtt              39

<210>   17
<211>   28
<212>   DNA
<213>   Antisense primer

<400>   17
tcttggatat cgaaagtttt accttgac                          28

<210>   18
<211>   28
<212>   DNA
<213>   Sense primer

<400>   18
gtcaaggtaa aactttcgat atccaaga                          28

<210>   19
<211>   39
<212>   DNA
<213>   Antisense primer

<400>   19
cataaaaaat caattttagt acatttggat attggcacc              39

<210>   20
<211>   37
<212>   DNA
<213>   Sense primer

<400>   20
atccaaatgt actaaaattg attttttatg acacttg                37

<210>   21
<211>   34
<212>   DNA
<213>   Antisense primer

<400>   21
tttatctaat gccattctag ttaaggcaat tgat                   34

<210>   22
<211>   35
<212>   DNA
<213>   Sense primer

<400>   22
gccttaacta gaatggcatt agataaatta gattt                  35
```

```
<210>  23
<211>  26
<212>  DNA
<213>  Antisense primer

<400>  23
aagtggaatc taaagctttt aattcg                                    26


<210>  24
<211>  26
<212>  DNA
<213>  Sense primer

<400>  24
cgaattaaaa gctttagatt ccactt                                    26


<210>  25
<211>  36
<212>  DNA
<213>  Antisense primer

<400>  25
cataaaaaat caattctacc aaacatcttc ttggta                         36


<210>  26
<211>  37
<212>  DNA
<213>  Sense primer

<400>  26
gaagatgttt ggtagaattg attttttatg acacttg                        37


<210>  27
<211>  24
<212>  DNA
<213>  Sense primer

<400>  27
gggtcacgga tccaatgttg ctgg                                      24


<210>  28
<211>  36
<212>  DNA
<213>  Antisense primer

<400>  28
gcagcagtgt atggatcctt agtgttcttt ggtggg                         36


<210>  29
<211>  24
<212>  DNA
<213>  Sense primer

<400>  29
gactttgatc aattttggta ccat                                      24


<210>  30
<211>  33
<212>  DNA
<213>  Antisense primer

<400>  30
agggtaccat gaagttttag actcttgatc act                            33
```

```
<210>  31
<211>  31
<212>  DNA
<213>  Sense primer

<400>  31
cttcttcaaa ccttcatatg acattgtttc g                    31

<210>  32
<211>  28
<212>  DNA
<213>  Antisense primer

<400>  32
ctaatggtca agcatatgtt gcattatc                        28

<210>  33
<211>  31
<212>  DNA
<213>  Sense primer

<400>  33
tttggttgac tcatatgtga gcgcggtaaa g                    31

<210>  34
<211>  31
<212>  DNA
<213>  Antisense primer

<400>  34
gttttgtctg gccatatgtt gaactggatg g                    31
```